# EUROPEAN PATENT SPECIFICATION

(11) **EP 3 361 259 B1**
(45) Date of publication and mention of the grant of the patent: **20.05.2020**
(21) Application number: 18161582.4
(22) Date of filing: 01.08.2014
(51) Int. Cl.: G01N 33/68

(54) **METHOD FOR PROGNOSIS OF THE EFFICACY OF ORAL IMMUNOTHERAPY FOR THE TREATMENT OF ALLERGY TO PROTEINS IN COW'S MILK**
VERFAHREN ZUR PROGNOSE DER WIRKSAMKEIT EINER ORALEN IMMUNTHERAPIE ZUR BEHANDLUNG VON ALLERGIEN AUF PROTEINE IN KUHMILCH
PROCÉDÉ DE PRONOSTIC DE L'EFFICACITÉ DE L'IMMUNOTHÉRAPIE ORALE POUR LE TRAITEMENT D'UNE ALLERGIE AUX PROTÉINES DU LAIT DE VACHE

(30) Priority: 01.08.2013 ES 201331200
(43) Date of publication of application: 15.08.2018
(62) Divisional of application: 14832504.6
(73) Proprietor: Fundación para la Investigación Biomédica del Hospital Universitario Ramón y Cajal, 28034 Madrid (ES); Fundación Para La Investigación Biomédica Del Hospital Clínico San Carlos, 28040 Madrid (ES)
(72) Inventor: DE LA HOZ CABALLER, Mª Belén, 28034 Madrid (ES); MARTÍNEZ BOTAS, Javier, 28034 Madrid (ES); CERECEDO CARBALLO, Inmaculada, 28034 Madrid (ES); FERNÁNDEZ RIVAS, Montserrat, 28040 Madrid (ES); RODRÍGUEZ ÁLVAREZ, Mónica, 28040 Madrid (ES)
(74) Representative: Hoffmann Eitle

(56) References cited:
- WO-A2-2012/137180
- WANG J ET AL: "Correlation of IgE/IgG4 milk epitopes and affinity of milk-specific IgE antibodies with different phenotypes of clinical milk allergy", JOURNAL OF ALLERGY AND CLINICAL IMMUNOLOGY, ELSEVIER, AMSTERDAM, NL, vol. 125, no. 3, 1 March 2010 (2010-03-01) , pages 695-702.e6, XP026942667, ISSN: 0091-6749, DOI: 10.1016/J.JACI.2009.12.017 [retrieved on 2010-03-10]
- Inmaculada Cerecedo Carballo ET AL: "Aplicación de la tecnología microarray al manejo diagnóstico de la alergia a proteínas de leche de vaca", , 1 January 2009 (2009-01-01), XP055317718, Retrieved from the Internet: URL:http://dspace.uah.es/dspace/bitstream/ handle/10017/6422/Versi?n final 200905024.pdf?sequence=1&isAllowed=y
- J. MARTÍNEZ-BOTAS ET AL: "Identification of novel peptide biomarkers to predict safety and efficacy of cow's milk oral immunotherapy by peptide microarray", CLINICAL & EXPERIMENTAL ALLERGY : JOURNAL OF THE BRITISH SOCIETY FOR ALLERGY AND CLINICAL IMMUNOLOGY, vol. 45, no. 6, 16 May 2015 (2015-05-16), pages 1071-1084, XP055341172, UK ISSN: 0954-7894, DOI: 10.1111/cea.12528

### SEQUENCE LISTING

<110> FUNDACIÓN PARA LA INVESTIGACIÓN BIOMÉDICA DEL HOSPITAL
   UNIVERSITARIO RAMON Y CAJAL
   FUNDACIÓN PARA LA INVESTIGACIÓN BIOMÉDICA DEL HOSPITAL
   CLINICO SAN CARLOS
<120> Method for prognosis of the efficacy of oral immunotherapy for the treatment of allergy to proteins in cow\222s milk.
<130> PT0042/2013
<160> 75
<170> Patent In version 3.5
<210> 1
   <211> 35
   <212> PRT
   <213> Bos taurus
<400> 1
   Leu Asn Glu Asn Leu Leu Arg Phe Phe Val Ala Pro Phe Pro Glu Val
   1               5                   10                  15
   Phe Gly Lys Glu Lys Val Asn Glu Leu Ser Lys Asp Ile Gly Ser Glu
               20                  25                  30
   Ser Thr Glu
           35
<210> 2
   <211> 56
   <212> PRT
   <213> Bos taurus
<400> 2
   Pro Asn Ser Val Glu Gln Lys His Ile Gln Lys Glu Asp Val Pro Ser
   1               5                   10                  15
   Glu Arg Tyr Leu Gly Tyr Leu Glu Gln Leu Leu Arg Leu Lys Lys Tyr
               20                  25                  30
   Lys Val Pro Gln Leu Glu Ile Val Pro Asn Ser Ala Glu Glu Arg Leu
           35                  40                  45
   His Ser Met Lys Glu Gly Ile His
       50                  55
<210> 3
   <211> 46
   <212> PRT
   <213> Bos taurus
<400> 3
   Ile His Ala Gln Gln Lys Glu Pro Met Ile Gly Val Asn Gln Glu Leu
   1               5                   10                  15
   Ala Tyr Phe Tyr Pro Glu Leu Phe Arg Gln Phe Tyr Gln Leu Asp Ala
               20                  25                  30
   Tyr Pro Ser Gly Ala Trp Tyr Tyr Val Pro Leu Gly Thr Gln
           35                  40                  45
<210> 4
   <211> 23
   <212> PRT
   <213> Bos taurus
<400> 4
   Asp Ala Pro Ser Phe Ser Asp Ile Pro Asn Pro Ile Gly Ser Glu Asn
   1               5                   10                  15
   Ser Glu Lys Thr Thr Met Pro
               20
<210> 5
   <211> 41
   <212> PRT
   <213> Bos taurus
<400> 5
   Thr Tyr Lys Gln Glu Lys Asn Met Ala Ile Asn Pro Ser Lys Glu Asn
   1               5                   10                  15
   Leu Cys Ser Thr Phe Cys Lys Glu Val Val Arg Asn Ala Asn Glu Glu
               20                  25                  30
   Glu Tyr Ser Ile Gly Ser Ser Ser Glu
           35                  40
<210> 6
   <211> 29
   <212> PRT
   <213> Bos taurus
<400> 6
   Ser Ala Glu Val Ala Thr Glu Glu Val Lys Ile Thr Val Asp Asp Lys
   1               5                   10                  15
   His Tyr Gln Lys Ala Leu Asn Glu Ile Asn Gln Phe Tyr
               20                  25
<210> 7
   <211> 41
   <212> PRT
   <213> Bos taurus
<400> 7
   Leu Asn Glu Ile Asn Gln Phe Tyr Gln Lys Phe Pro Gln Tyr Leu Gln
   1               5                   10                  15
   Tyr Leu Tyr Gln Gly Pro Ile Val Leu Asn Pro Trp Asp Gln Val Lys
               20                  25                  30
   Arg Asn Ala Val Pro Ile Thr Pro Thr
           35                  40
<210> 8
   <211> 26
   <212> PRT
   <213> Bos taurus
<400> 8
   Thr Lys Lys Thr Lys Leu Thr Glu Glu Glu Lys Asn Arg Leu Asn Phe
   1               5                   10                  15
   Leu Lys Lys Ile Ser Gln Arg Tyr Gln Lys
               20                  25
<210> 9
   <211> 20
   <212> PRT
   <213> Bos taurus
<400> 9
   Arg Glu Leu Glu Glu Leu Asn Val Pro Gly Glu Ile Val Glu Ser Leu
   1               5                   10                  15
   Ser Ser Ser Glu
               20
<210> 10
   <211> 20
   <212> PRT
   <213> Bos taurus
<400> 10
   Asp Glu Leu Gln Asp Lys Ile His Pro Phe Ala Gln Thr Gln Ser Leu
   1               5                   10                  15
   Val Tyr Pro Phe
               20
<210> 11
   <211> 29
   <212> PRT
   <213> Bos taurus
<400> 11
   Asn Ile Pro Pro Leu Thr Gln Thr Pro Val Val Val Pro Pro Phe Leu
   1               5                   10                  15
   Gln Pro Glu Val Met Gly Val Ser Lys Val Lys Glu Ala
               20                  25
<210> 12
   <211> 32
   <212> PRT
   <213> Bos taurus
<400> 12
   Gly Val Ser Lys Val Lys Glu Ala Met Ala Pro Lys His Lys Glu Met
   1               5                   10                  15
   Pro Phe Pro Lys Tyr Pro Val Glu Pro Phe Thr Glu Ser Gln Ser Leu
               20                  25                  30
<210> 13
   <211> 83
   <212> **PRT**
   <213> Bos taurus
<400> 13 Ile Ile Val
<210> 14
   <211> 35
   <212> PRT
   <213> Bos taurus
<400> 14
   Gln Pro Ile Arg Cys Glu Lys Asp Glu Arg Phe Phe Ser Asp Lys Ile
   1               5                   10                  15
   Ala Lys Tyr Ile Pro Ile Gln Tyr Val Leu Ser Arg Tyr Pro Ser Tyr
               20                  25                  30
   Gly Leu Asn
           35
<210> 15
   <211> 23
   <212> PRT
   <213> Bos taurus
<400> 15
   Ala Leu Ile Asn Asn Gln Phe Leu Pro Tyr Pro Tyr Tyr Ala Lys Pro
   1               5                   10                  15
   Ala Ala Val Arg Ser Pro Ala
               20
<210> 16
   <211> 20
   <212> PRT
   <213> Bos taurus
<400> 16
   Ala Gly Thr Trp Tyr Ser Leu Ala Met Ala Ala Ser Asp Ile Ser Leu
   1               5                   10                  15
   Leu Asp Ala Gln
               20
<210> 17
   <211> 35
   <212> PRT
   <213> Bos taurus
<400> 17
   Arg Thr Pro Glu Val Asp Asp Glu Ala Leu Glu Lys Phe Asp Lys Ala
   1               5                   10                  15
   Leu Lys Ala Leu Pro Met His Ile Arg Leu Ser Phe Asn Pro Thr Gln
               20                  25                  30
   Leu Glu Glu
           35
<210> 18
   <211> 20
   <212> PRT
   <213> Bos taurus
<400> 18
   Leu Asn Glu Asn Leu Leu Arg Phe Phe Val Ala Pro Phe Pro Glu Val
   1               5                   10                  15
   Phe Gly Lys Glu
               20
<210> 19
   <211> 20
   <212> PRT
   <213> Bos taurus
<400> 19
   Val Ala Pro Phe Pro Glu Val Phe Gly Lys Glu Lys Val Asn Glu Leu
   1               5                   10                  15
   Ser Lys Asp Ile
               20
<210> 20
   <211> 20
   <212> PRT
   <213> Bos taurus
<400> 20
   Val Phe Gly Lys Glu Lys Val Asn Glu Leu Ser Lys Asp Ile Gly Ser
   1               5                   10                  15
   Glu Ser Thr Glu
               20
<210> 21
   <211> 20
   <212> PRT
   <213> Bos taurus
<400> 21
   Pro Asn Ser Val Glu Gln Lys His Ile Gln Lys Glu Asp Val Pro Ser
   1               5                   10                  15
   Glu Arg Tyr Leu
               20
<210> 22
   <211> 20
   <212> PRT
   <213> Bos taurus
<400> 22
   Gln Lys Glu Asp Val Pro Ser Glu Arg Tyr Leu Gly Tyr Leu Glu Gln
   1               5                   10                  15
   Leu Leu Arg Leu
               20
<210> 23
   <211> 20
   <212> PRT
   <213> Bos taurus
<400> 23
   Tyr Leu Gly Tyr Leu Glu Gln Leu Leu Arg Leu Lys Lys Tyr Lys Val
   1               5                   10                  15
   Pro Gln Leu Glu
               20
<210> 24
   <211> 20
   <212> PRT
   <213> Bos taurus
<400> 24
   Arg Leu Lys Lys Tyr Lys Val Pro Gln Leu Glu Ile Val Pro Asn Ser
   1               5                   10                  15
   Ala Glu Glu Arg
               20
<210> 25
   <211> 20
   <212> PRT
   <213> Bos taurus
<400> 25
   Leu Glu Ile Val Pro Asn Ser Ala Glu Glu Arg Leu His Ser Met Lys
   1               5                   10                  15
   Glu Gly Ile His
               20
<210> 26
   <211> 20
   <212> PRT
   <213> Bos taurus
<400> 26
   Ile His Ala Gln Gln Lys Glu Pro Met Ile Gly Val Asn Gln Glu Leu
   1               5                   10                  15
   Ala Tyr Phe Tyr
               20
<210> 27
   <211> 20
   <212> PRT
   <213> Bos taurus
<400> 27
   Glu Pro Met Ile Gly Val Asn Gln Glu Leu Ala Tyr Phe Tyr Pro Glu
   1               5                   10                  15
   Leu Phe Arg Gln
               20
<210> 28
   <211> 20
   <212> PRT
   <213> Bos taurus
<400> 28
   Ile Gly Val Asn Gln Glu Leu Ala Tyr Phe Tyr Pro Glu Leu Phe Arg
   1               5                   10                  15
   Gln Phe Tyr Gln
               20
<210> 29
   <211> 20
   <212> PRT
   <213> Bos taurus
<400> 29
   Phe Tyr Pro Glu Leu Phe Arg Gln Phe Tyr Gln Leu Asp Ala Tyr Pro
   1               5                   10                  15
   Ser Gly Ala Trp
               20
<210> 30
   <211> 20
   <212> PRT
   <213> Bos taurus
<400> 30
   Tyr Gln Leu Asp Ala Tyr Pro Ser Gly Ala Trp Tyr Tyr Val Pro Leu
   1 5 10 15
   Gly Thr Gln Tyr
   20
<210> 31
   <211> 20
   <212> PRT
   <213> Bos taurus
<400> 31
   Asp Ala Pro Ser Phe Ser Asp Ile Pro Asn Pro Ile Gly Ser Glu Asn
   1 5 10 15
   Ser Glu Lys Thr
   20
<210> 32
   <211> 20
   <212> PRT
   <213> Bos taurus
<400> 32
   Ser Phe Ser Asp Ile Pro Asn Pro Ile Gly Ser Glu Asn Ser Glu Lys
   1 5 10 15
   Thr Thr Met Pro
   20
<210> 33
   <211> 20
   <212> PRT
   <213> Bos taurus
<400> 33
   Thr Tyr Lys Gln Glu Lys Asn Met Ala Ile Asn Pro Ser Lys Glu Asn
   1 5 10 15
   Leu Cys Ser Thr
   20
<210> 34
   <211> 20
   <212> PRT
   <213> Bos taurus
<400> 34
   Asn Met Ala Ile Asn Pro Ser Lys Glu Asn Leu Cys Ser Thr Phe Cys
   1 5 10 15
   Lys Glu Val Val
   20
<210> 35
   <211> 20
   <212> PRT
   <213> Bos taurus
<400> 35
   Ser Lys Glu Asn Leu Cys Ser Thr Phe Cys Lys Glu Val Val Arg Asn
   1 5 10 15
   Ala Asn Glu Glu
   20
<210> 36
   <211> 20
   <212> PRT
   <213> Bos taurus
<400> 36
   Cys Lys Glu Val Val Arg Asn Ala Asn Glu Glu Glu Tyr Ser Ile Gly
   1 5 10 15
   Ser Ser Ser Glu
   20
<210> 37
   <211> 20
   <212> PRT
   <213> Bos taurus
<400> 37
   Ser Ala Glu Val Ala Thr Glu Glu Val Lys Ile Thr Val Asp Asp Lys
   1 5 10 15
   His Tyr Gln Lys
   20
<210> 38
   <211> 20
   <212> PRT
   <213> Bos taurus
<400> 38
   Val Ala Thr Glu Glu Val Lys Ile Thr Val Asp Asp Lys His Tyr Gln
   1 5 10 15
   Lys Ala Leu Asn
   20
<210> 39
   <211> 20
   <212> PRT
   <213> Bos taurus
<400> 39
   Glu Glu Val Lys Ile Thr Val Asp Asp Lys His Tyr Gln Lys Ala Leu
   1 5 10 15
   Asn Glu Ile Asn
   20
<210> 40
   <211> 20
   <212> PRT
   <213> Bos taurus
<400> 40
   Lys Ile Thr Val Asp Asp Lys His Tyr Gln Lys Ala Leu Asn Glu Ile
   1 5 10 15
   Asn Gln Phe Tyr
   20
<210> 41
   <211> 20
   <212> PRT
   <213> Bos taurus
<400> 41
   Leu Asn Glu Ile Asn Gln Phe Tyr Gln Lys Phe Pro Gln Tyr Leu Gln
   1 5 10 15
   Tyr Leu Tyr Gln
   20
<210> 42
   <211> 20
   <212> PRT
   <213> Bos taurus
<400> 42
   Gln Tyr Leu Gln Tyr Leu Tyr Gln Gly Pro Ile Val Leu Asn Pro Trp
   1 5 10 15
   Asp Gln Val Lys
   20
<210> 43
   <211> 20
   <212> PRT
   <213> Bos taurus
<400> 43
   Pro Ile Val Leu Asn Pro Trp Asp Gln Val Lys Arg Asn Ala Val Pro
   1               5                   10                  15
   Ile Thr Pro Thr
               20
<210> 44
   <211> 20
   <212> PRT
   <213> Bos taurus
<400> 44
   Thr Lys Lys Thr Lys Leu Thr Glu Glu Glu Lys Asn Arg Leu Asn Phe
   1               5                   10                  15
   Leu Lys Lys Ile
               20
<210> 45
   <211> 20
   <212> PRT
   <213> Bos taurus
<400> 45
   Thr Glu Glu Glu Lys Asn Arg Leu Asn Phe Leu Lys Lys Ile Ser Gln
   1               5                   10                  15
   Arg Tyr Gln Lys
               20
<210> 46
   <211> 20
   <212> PRT
   <213> Bos taurus
<400> 46
   Arg Glu Leu Glu Glu Leu Asn Val Pro Gly Glu Ile Val Glu Ser Leu
   1               5                   10                  15
   Ser Ser Ser Glu
               20
<210> 47
   <211> 20
   <212> PRT
   <213> Bos taurus
<400> 47
   Asp Glu Leu Gln Asp Lys Ile His Pro Phe Ala Gln Thr Gln Ser Leu
   1               5                   10                  15
   Val Tyr Pro Phe
               20
<210> 48
   <211> 20
   <212> PRT
   <213> Bos taurus
<400> 48
   Asn Ile Pro Pro Leu Thr Gln Thr Pro Val Val Val Pro Pro Phe Leu
   1               5                   10                  15
   Gln Pro Glu Val
               20
<210> 49
   <211> 20
   <212> PRT
   <213> Bos taurus
<400> 49
   Val Val Val Pro Pro Phe Leu Gln Pro Glu Val Met Gly Val Ser Lys
   1               5                   10                  15
   Val Lys Glu Ala
               20
<210> 50
   <211> 20
   <212> PRT
   <213> Bos taurus
<400> 50
   Gly Val Ser Lys Val Lys Glu Ala Met Ala Pro Lys His Lys Glu Met
   1               5                   10                  15
   Pro Phe Pro Lys
               20
<210> 51
   <211> 20
   <212> PRT
   <213> Bos taurus
<400> 51
   Glu Ala Met Ala Pro Lys His Lys Glu Met Pro Phe Pro Lys Tyr Pro
   1               5                   10                  15
   Val Glu Pro Phe
               20
<210> 52
   <211> 20
   <212> PRT
   <213> Bos taurus
<400> 52
   His Lys Glu Met Pro Phe Pro Lys Tyr Pro Val Glu Pro Phe Thr Glu
   1               5                   10                  15
   Ser Gln Ser Leu
               20
<210> 53
   <211> 20
   <212> PRT
   <213> Bos taurus
<400> 53
   Leu Thr Asp Val Glu Asn Leu His Leu Pro Leu Pro Leu Leu Gln Ser
   1               5                   10                  15
   Trp Met His Gln
               20
<210> 54
   <211> 20
   <212> PRT
   <213> Bos taurus
<400> 54
   Leu His Leu Pro Leu Pro Leu Leu Gln Ser Trp Met His Gln Pro His
   1               5                   10                  15
   Gln Pro Leu Pro
               20
<210> 55
   <211> 20
   <212> PRT
   <213> Bos taurus
<400> 55
   Leu Leu Gln Ser Trp Met His Gln Pro His Gln Pro Leu Pro Pro Thr
<210> 56
   <211> 20
   <212> PRT
   <213> Bos taurus
<400> 56
   Ser Trp Met His Gln Pro His Gln Pro Leu Pro Pro Thr Val Met Phe
   1 5 10 15
   Pro Pro Gln Ser
   20
<210> 57
   <211> 20
   <212> PRT
   <213> Bos taurus
<400> 57
   Leu Pro Pro Thr Val Met Phe Pro Pro Gln Ser Val Leu Ser Leu Ser
   1 5 10 15
   Gln Ser Lys Val
   20
<210> 58
   <211> 20
   <212> PRT
   <213> Bos taurus
<400> 58
   Phe Pro Pro Gln Ser Val Leu Ser Leu Ser Gln Ser Lys Val Leu Pro
   1 5 10 15
   Val Pro Gln Lys
   20
<210> 59
   <211> 20
   <212> PRT
   <213> Bos taurus
<400> 59
   Gln Ser Val Leu Ser Leu Ser Gln Ser Lys Val Leu Pro Val Pro Gln
   1 5 10 15
   Lys Ala Val Pro
               20
<210> 60
   <211> 20
   <212> PRT
   <213> Bos taurus
<400> 60
   Ser Gln Ser Lys Val Leu Pro Val Pro Gln Lys Ala Val Pro Tyr Pro
   1               5                   10                  15
   Gln Arg Asp Met
               20
<210> 61
   <211> 20
   <212> PRT
   <213> Bos taurus
<400> 61
   Gln Lys Ala Val Pro Tyr Pro Gln Arg Asp Met Pro Ile Gln Ala Phe
   1               5                   10                  15
   Leu Leu Tyr Gln
               20
<210> 62
   <211> 20
   <212> PRT
   <213> Bos taurus
<400> 62
   Pro Gln Arg Asp Met Pro Ile Gln Ala Phe Leu Leu Tyr Gln Glu Pro
   1               5                   10                  15
   Val Leu Gly Pro
               20
<210> 63
   <211> 20
   <212> PRT
   <213> Bos taurus
<400> 63
   Phe Leu Leu Tyr Gln Glu Pro Val Leu Gly Pro Val Arg Gly Pro Phe
   1               5                   10                  15
   Pro Ile Ile Val
               20
<210> 64
   <211> 20
   <212> PRT
   <213> Bos taurus
<400> 64
   Gln Pro Ile Arg Cys Glu Lys Asp Glu Arg Phe Phe Ser Asp Lys Ile
   1               5                   10                  15
   Ala Lys Tyr Ile
               20
<210> 65
   <211> 20
   <212> PRT
   <213> Bos taurus
<400> 65
   Lys Asp Glu Arg Phe Phe Ser Asp Lys Ile Ala Lys Tyr Ile Pro Ile
   1               5                   10                  15
   Gln Tyr Val Leu
               20
<210> 66
   <211> 20
   <212> PRT
   <213> Bos taurus
<400> 66
   Ser Asp Lys Ile Ala Lys Tyr Ile Pro Ile Gln Tyr Val Leu Ser Arg
   1               5                   10                  15
   Tyr Pro Ser Tyr
               20
<210> 67
   <211> 20
   <212> PRT
   <213> Bos taurus
<400> 67
   Ile Ala Lys Tyr Ile Pro Ile Gln Tyr Val Leu Ser Arg Tyr Pro Ser
   1               5                   10                  15
   Tyr Gly Leu Asn
               20
<210> 68
   <211> 20
   <212> PRT
   <213> Bos taurus
<400> 68
   Ala Leu Ile Asn Asn Gln Phe Leu Pro Tyr Pro Tyr Tyr Ala Lys Pro
   1               5                   10                  15
   Ala Ala Val Arg
               20
<210> 69
   <211> 20
   <212> PRT
   <213> Bos taurus
<400> 69
   Asn Asn Gln Phe Leu Pro Tyr Pro Tyr Tyr Ala Lys Pro Ala Ala Val
   1               5                   10                  15
   Arg Ser Pro Ala
               20
<210> 70
   <211> 20
   <212> PRT
   <213> Bos taurus
<400> 70
   Ala Gly Thr Trp Tyr Ser Leu Ala Met Ala Ala Ser Asp Ile Ser Leu
   1               5                   10                  15
   Leu Asp Ala Gln
               20
<210> 71
   <211> 20
   <212> PRT
   <213> Bos taurus
<400> 71
   Arg Thr Pro Glu Val Asp Asp Glu Ala Leu Glu Lys Phe Asp Lys Ala
   1               5                   10                  15
   Leu Lys Ala Leu
               20
<210> 72
   <211> 20
   <212> PRT
   <213> Bos taurus
<400> 72
   Glu Val Asp Asp Glu Ala Leu Glu Lys Phe Asp Lys Ala Leu Lys Ala
   1               5                   10                  15
   Leu Pro Met His
               20
<210> 73
   <211> 20
   <212> PRT
   <213> Bos taurus
<400> 73
   Asp Glu Ala Leu Glu Lys Phe Asp Lys Ala Leu Lys Ala Leu Pro Met
   1               5                   10                  15
   His Ile Arg Leu
               20
<210> 74
   <211> 20
   <212> PRT
   <213> Bos taurus
<400> 74
   Phe Asp Lys Ala Leu Lys Ala Leu Pro Met His Ile Arg Leu Ser Phe
   1               5                   10                  15
   Asn Pro Thr Gln
               20
<210> 75
   <211> 20
   <212> PRT
   <213> Bos taurus
<400> 75
   Ala Leu Lys Ala Leu Pro Met His Ile Arg Leu Ser Phe Asn Pro Thr
   1               5                   10                  15
   Gln Leu Glu Glu
               20

## Description

### FIELD OF THE INVENTION

The present invention is comprised in the general field of allergology and relates particularly to a method for prognosis of the efficacy of the treatment for desensitization to cow's milk.

### PRIOR ART

Food allergy with immediate hypersensitivity does not have an established effective treatment today, the only alternative for the patients is to avoid same until spontaneous tolerance to said food is achieved, a situation achieved by a large number of patients but not by all.

Allergy to proteins in cow's milk (APCW) occurs when an individual, after consuming dairy products, shows an abnormal response (with symptoms that can be included in adverse reactions to food) and when there is a proven immunological mechanism in that process.

Proteins responsible for allergenicity in cow's milk are caseins (alpha-casein, beta-casein and kappa-casein) and beta-lactoglobulin, alpha-lactalbumin, bovine serum albumin and bovine immunoglobulins serum proteins as well as other proteins at a lower proportion: lactoferrin, transferrin, lipase which are serum proteins and represent 2% of the total protein in whole cow's milk.

Caseins are the main cause of allergy to cow's milk.

It has recently been proven that there are different clinical phenotypes of reaction severity in food allergy. Although several factors have been involved in these differences, the geographical component plays a very significant role. A clear example is fruit allergy in which individuals who are allergic to a certain food because they recognize different allergens (whole protein/peptide) have different clinical severity. Therefore, individuals who are allergic to peaches from the north of Europe only have mild symptoms after consuming them, while patients from the south of Europe have severe systemic reactions. Knowing the recognition pattern of allergens of a specific food, both the whole proteins and ideally the epitopes thereof, which a specific population has, is therefore of greatest interest.

There are different methods today for diagnosing allergy to cow's milk, but as a norm, detection of IgE specific to different proteins in cow's milk is performed after the first allergic attack.

Oral immunotherapy (OIT) with food is a novel therapeutic method the objective of which is to achieve in patients allergic to a specific food tolerance to same by means of the continuous administration of said food starting from minimal doses until reaching tolerance to a common amount of the food.

Cow's milk is a ubiquitous food that is very hard to avoid and produces severe reactions. In the last ten years, many research groups have developed protocols for the oral administration of cow's milk in order to achieve tolerance to same. In the current state of knowledge, the treatment achieves good efficacy levels as it successfully induces tolerance to cow's milk. Nevertheless, its biggest problem lies in the safety of the treatment since the patients show a high number of allergic reactions when performing the procedure. Although said allergic reactions are mild and easily treatable on most occasions, they are severe and even require the administration of adrenaline on very few occasions. Therefore, reactions during the procedure are one of the main difficulties when it comes to incorporating the procedure into a common practice for the disease, and therefore expanding the use thereof to a significant number of patients.

There is therefore a need to provide a method for prognosis of the efficacy of the treatment for desensitization to cow's milk and therefore of the patient response to same.

### DISCLOSURE OF THE INVENTION

The present invention provides a solution to the problems stated in the state of the art since it provides a method which allows making a prognosis of the number of reactions that will be produced during oral immunotherapy (OIT) against proteins in cow's milk and/or an estimate of the treatment time required to achieve tolerance or desensitization during OIT against proteins in cow's milk and/or the need for pre-medication during OIT in a human subject.

The invention is defined in the appended claims.

Therefore, a first aspect of the invention relates to a method for prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT in a human subject, comprising the following steps:
a. Isolating a biological sample from the subject;
b. Determining the presence or absence of IgE antibodies in the biological sample of step a) against or with specificity for peptides identified with the following sequences:
   a. At least one sequence selected from the group consisting of peptide sequences SEQ ID NO: 1-4 and/or fragments thereof selected from the list consisting of sequences SEQ ID NO: 18-32 and/or derivatives of sequences SEQ ID NO: 18-32 that are immunologically active; and/or
   b. At least one sequence selected from the group consisting of peptide sequences SEQ ID NO: 5-8 and/or fragments thereof selected from the list consisting of sequences SEQ ID NO: 33-45 and/or derivatives of sequences SEQ ID NO: 33-45 that are immunologically active; and/or
   c. At least one sequence selected from the group consisting of peptide sequences SEQ ID NO: 9-13 and/or fragments thereof selected from the list consisting of sequences SEQ ID NO: 46-63 and/or derivatives of sequences SEQ ID NO: 46-63 that are immunologically active; and/or
   d. At least one sequence selected from the group consisting of peptide sequences SEQ ID NO: 14-15 and/or fragments thereof selected from the list consisting of sequences SEQ ID NO: 64-69 and/or derivatives of sequences SEQ ID NO: 64-69 that are immunologically active; and/or
   e. At least one sequence selected from the group consisting of peptide sequences SEQ ID NO: 16-17 and/or fragments thereof selected from the list consisting of sequences SEQ ID NO: 70-75 and/or derivatives of sequences SEQ ID NO: 70-75 that are immunologically active;
where the prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment.

A preferred embodiment of the first aspect of the invention relates to a method for prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT in a human subject, comprising the following steps:
a. Isolating a biological sample from the subject;
b. Determining the presence or absence of IgE antibodies in the biological sample of step a) against or with specificity for peptides identified with the following sequences:
   a. At least one sequence selected from the group consisting of peptide sequences SEQ ID NO: 1-4 and/or fragments thereof selected from the list consisting of sequences SEQ ID NO: 18-32 and/or derivatives of sequences SEQ ID NO: 18-32 that are immunologically active; and
   b. At least one sequence selected from the group consisting of peptide sequences SEQ ID NO: 5-8 and/or fragments thereof selected from the list consisting of sequences SEQ ID NO: 33-45 and/or derivatives of sequences SEQ ID NO: 33-45 that are immunologically active; and
   c. At least one sequence selected from the group consisting of peptide sequences SEQ ID NO: 9-13 and/or fragments thereof selected from the list consisting of sequences SEQ ID NO: 46-63 and/or derivatives of sequences SEQ ID NO: 46-63 that are immunologically active; and optionally
   d. At least one sequence selected from the group consisting of peptide sequences SEQ ID NO: 14-15 and/or fragments thereof selected from the list consisting of sequences SEQ ID NO: 64-69 and/or derivatives of sequences SEQ ID NO: 64-69 that are immunologically active; and optionally
   e. At least one sequence selected from the group consisting of peptide sequences SEQ ID NO: 16-17 and/or fragments thereof selected from the list consisting of sequences SEQ ID NO: 70-75 and/or derivatives of sequences SEQ ID NO: 70-75 that are immunologically active;
where the prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment.

Another preferred embodiment of the first aspect of the invention relates to a method for prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT in a human subject, comprising the following steps:
a. Isolating a biological sample from the subject;
b. Determining the presence or absence of IgE antibodies in the biological sample of step a) against or with specificity for peptides identified with the following sequences:
   a. At least one sequence selected from the group consisting of peptide sequences SEQ ID NO: 1-4 and/or fragments thereof selected from the list consisting of sequences SEQ ID NO: 18-32 and/or derivatives of sequences SEQ ID NO: 18-32 that are immunologically active; and
   b. At least one sequence selected from the group consisting of peptide sequences SEQ ID NO: 5-8 and/or fragments thereof selected from the list consisting of sequences SEQ ID NO: 33-45 and/or derivatives of sequences SEQ ID NO: 33-45 that are immunologically active; and
   c. At least one sequence selected from the group consisting of peptide sequences SEQ ID NO: 9-13 and/or fragments thereof selected from the list consisting of sequences SEQ ID NO: 46-63 and/or derivatives of sequences SEQ ID NO: 46-63 that are immunologically active; and
   d. At least one sequence selected from the group consisting of peptide sequences SEQ ID NO: 14-15 and/or fragments thereof selected from the list consisting of sequences SEQ ID NO: 64-69 and/or derivatives of sequences SEQ ID NO: 64-69 that are immunologically active; and
   e. At least one sequence selected from the group consisting of peptide sequences SEQ ID NO: 16-17 and/or fragments thereof selected from the list consisting of sequences SEQ ID NO: 70-75 and/or derivatives of sequences SEQ ID NO: 70-75 that are immunologically active;
where the prognosis of the number of reactions during oral immunotherapy (OIT) against proteins in cow's milk and/or an estimate of the treatment time required to achieve tolerance or desensitization during OIT against proteins in cow's milk and/or the need for pre-medication during OIT is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment.

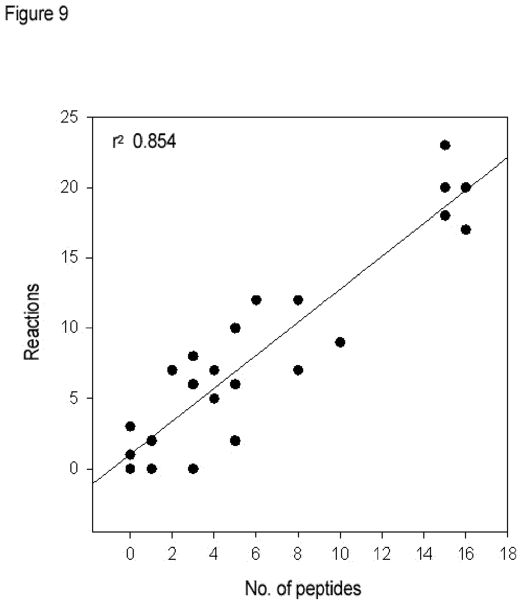

In another preferred embodiment of the first aspect of the invention, the method is a method for prognosis of the number of reactions during oral immunotherapy (OIT) and the determination of the presence or absence of IgE antibodies of step b) in the biological sample of step a) is performed by determining the presence or absence of IgE antibodies against or with specificity for at least the following peptides identified with the following sequences:
a. sequences SEQ ID NO: 19, 21, 25 and 27 and/or derivatives of sequences SEQ ID NO: 19, 21, 25 and 27 that are immunologically active; and
b. sequences SEQ ID NO: 36, 37 and 38 and/or derivatives of sequences SEQ ID NO: 36, 37 and 38 that are immunologically active; and
c. sequences SEQ ID NO: 51, 53, 58, 59 and 62 and/or derivatives of sequences SEQ ID NO: 51, 53, 58, 59 and 62 that are immunologically active; and
d. sequence SEQ ID NO: 69 and/or a derivative of the sequence SEQ ID NO: 69 that is immunologically active; and
e. sequences SEQ ID NO: 71 and 72 and/or derivatives of sequences SEQ ID NO: 71 and 72 that are immunologically active;
where the prognosis of the number of reactions during oral immunotherapy (OIT) against proteins in cow's milk is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment. Preferably, the reference values are established in Figure 9.

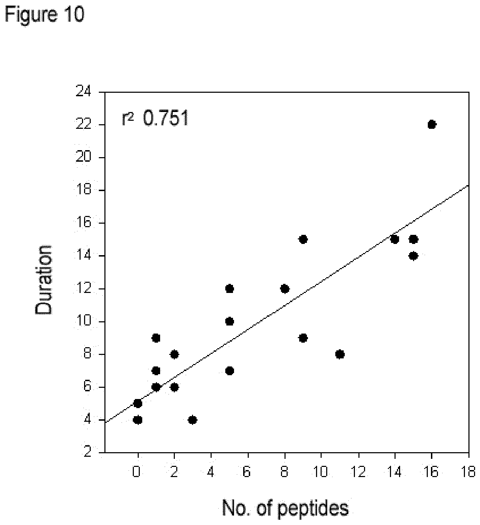

In another preferred embodiment of the first aspect of the invention, the method is a method for prognosis of an estimate of the treatment time required to achieve tolerance or desensitization during OIT and the determination of the presence or absence of IgE antibodies of step b) in the biological sample of step a) is performed by determining the presence or absence of IgE antibodies against or with specificity for at least the following peptides identified with the following sequences:
a. sequences SEQ ID NO: 28 and 31 and/or derivatives of sequences SEQ ID NO: 28 and 31 that are immunologically active; and
b. sequences SEQ ID NO: 36-40 and 44 and/or derivatives of sequences SEQ ID NO: 36-40 and 44 that are immunologically active; and
c. sequences SEQ ID NO: 53, 55 and 56 and/or derivatives of sequences SEQ ID NO: 53, 55 and 56 that are immunologically active; and
d. sequences SEQ ID NO: 67 and 68 and/or a derivative of the sequence SEQ ID NO: 67 and 68 that are immunologically active; and
e. sequences SEQ ID NO: 70, 72 and 75 and/or derivatives of sequences SEQ ID NO: 70, 72 and 75 that are immunologically active;
where an estimate of the treatment time required to achieve tolerance or desensitization during OIT against proteins in cow's milk is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment. Preferably, the reference values are established in Figure 10.

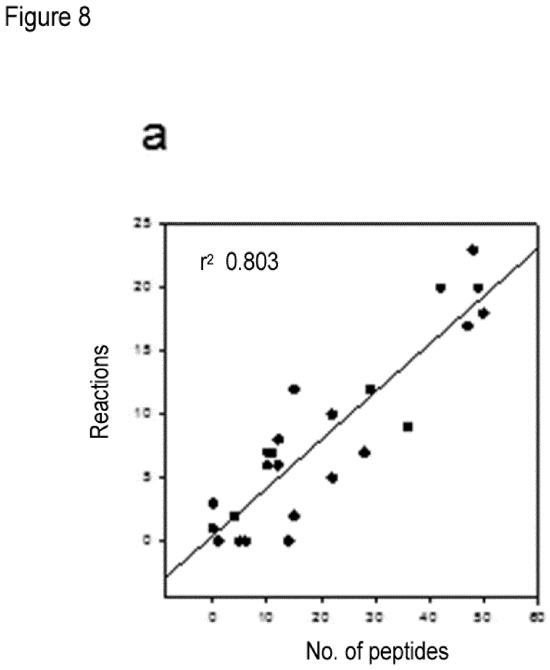

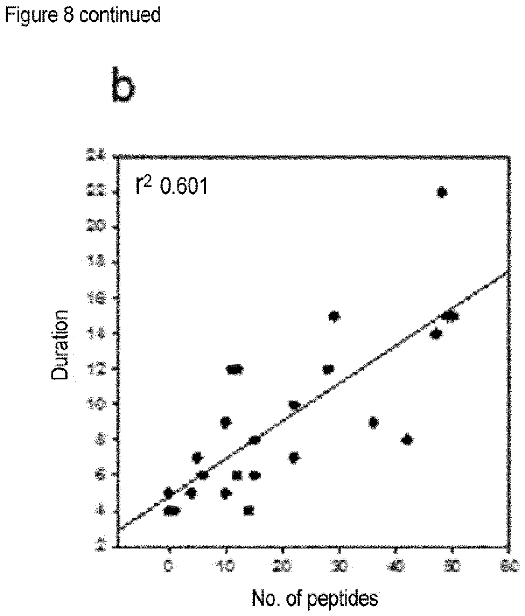

In yet another preferred embodiment of the first aspect of the invention, the determination of the presence or absence of IgE antibodies in the biological sample of step a) is performed by determining the presence or absence of IgE antibodies against or with specificity for at least the following peptides identified with the following sequences: SEQ ID NO: 18-75 and/or derivatives of sequences SEQ ID NO: 18-75 that are immunologically active, where the prognosis of the number of reactions during oral immunotherapy (OIT) against proteins in cow's milk is established by correlating the number of peptide sequences recognized by IgE antibodies in the samples from the subjects before starting OIT treatment (time 0) with reference values established based on those same peptide sequences which correlate the number of peptides recognized by IgE antibodies in the samples from the patients with the response after OIT treatment. Preferably, the reference values are established in Figure 8.

In yet another preferred embodiment of the first aspect of the invention, the determination of the presence or absence of IgE antibodies in the biological sample of step a) is performed by determining the presence or absence of IgE antibodies against or with specificity for at least the following peptides identified with the following sequences: SEQ ID NO: 18-75 and/or preferably derivatives of sequences SEQ ID NO: 18-75 that are immunologically active, where:
- a peptide recognition of less than 15% is indicative of a good prognosis,
- a peptide recognition of between 15%-75% is indicative of a moderate prognosis
- a peptide recognition of more than 75% is indicative of a poor prognosis.

In a preferred embodiment of the first aspect of the invention or of any of the preferred embodiments thereof, the biological sample includes different types of tissue samples, as well as biological fluid samples, such as blood, serum, plasma, cerebrospinal fluid, peritoneal fluid, feces or urine. The sample is preferably selected from blood, plasma, serum or urine.

In a preferred embodiment of the first aspect of the invention or of any of the preferred embodiments thereof, the presence of peptides is determined by means of an immunoassay.

In a preferred embodiment of the first aspect of the invention or of any of the preferred embodiments thereof, the presence of peptides is determined by means of peptide microarrays.

A second aspect of the invention relates to a peptide microarray suitable for carrying out the method of the first aspect of the invention or any of the preferred embodiments thereof, comprising the combinations of peptide sequences defined in said method or in said embodiments.

A third aspect relates to a composition suitable for carrying out the method of the first aspect of the invention or any of the preferred embodiments thereof, comprising the combinations of peptides defined in said method or in said embodiments.

A fourth aspect relates to a kit suitable for carrying out the method of the first aspect of the invention or any of the preferred embodiments thereof, comprising the combinations of peptides defined in said method or in said embodiments.

In a preferred embodiment of the fourth aspect of the invention, said kit comprises:
a. Recognition molecules (capture biomolecules) capable of recognizing IgE selected from the list consisting of the different combinations of peptide sequences defined in the method of the first aspect of the invention or in any of the preferred embodiments thereof; and
b. A second recognition molecule (detection biomolecule) capable of recognizing the target analyte or the capture biomolecule optionally bound to a tag molecule.

In another preferred embodiment of the fourth aspect of the invention, said kit comprises:
a. Recognition molecules (capture biomolecules) capable of recognizing IgE selected from the list consisting of the different combinations of peptides defined in the method of the first aspect of the invention or in any of the preferred embodiments thereof; and
b. A support where the recognition biomolecules of step a) are immobilized; and
c. A second recognition molecule (detection biomolecule) capable of recognizing the target analyte or the capture biomolecule optionally bound to a tag molecule.

A fifth aspect of the invention relates to the use of the kit of the fourth aspect of the invention or of any of the preferred embodiments thereof, or the composition of the third aspect of the invention or the peptide microarray of the second aspect of the invention, for prognosis of the number of reactions during oral immunotherapy (OIT) against proteins in cow's milk and/or an estimate of the treatment time required to achieve tolerance or desensitization during OIT and/or the need to administer pre-medication during OIT.

In a sixth aspect of the invention, the present invention relates to a method for prognosis and follow-up of the treatment of allergy to proteins in cow's milk with oral immunotherapy with cow's milk which comprises determining the presence of IgE or IgG4 antibodies against the peptides identified with peptide sequences SEQ ID NO: 1-17 and/or fragments thereof in a sample isolated from a subject.

In a particular embodiment of the sixth aspect of the invention, a peptide recognition of less than 15% (peptides recognized by IgE antibodies in the biological sample of step a) is indicative of a good prognosis.

In a particular embodiment of the sixth aspect of the invention, a peptide recognition comprised between 15%-75% (peptides recognized by IgE antibodies in the biological sample of step a) is indicative of a moderate prognosis.

In a particular embodiment of the sixth aspect of the invention, a peptide recognition of more than 75% (peptides recognized by IgE antibodies in the biological sample of step a) is indicative of a poor prognosis.

In a particular embodiment of the sixth aspect of the invention, the fragments of the peptides (those recognized by IgE antibodies in the biological sample of step a) are identified with sequences SEQ ID NO: 18-75.

In a particular embodiment of the sixth aspect of the invention, the sample to be analyzed is selected from blood, serum or urine.

In a particular embodiment of the sixth aspect of the invention, the presence of peptides and/or fragments thereof is determined by means of an immunoassay.

In a particular embodiment of the sixth aspect of the invention, the presence of peptides and/or fragments thereof is determined by means of peptide microarrays.

A seventh aspect of the invention relates to a microarray for prognosis and follow-up of the treatment of allergy to proteins in cow's milk with oral immunotherapy with cow's milk according to the method of the sixth aspect of the present invention comprising peptides of peptide sequences SEQ ID NO: 1-17 and/or fragments thereof.

A eighth aspect of the invention relates to a kit for prognosis and follow-up of the treatment of allergy to proteins in cow's milk with oral immunotherapy with cow's milk according to the method of the sixth aspect of the present invention, comprising peptides of peptide sequences SEQ ID NO: 1-17 and/or fragments thereof.

In another aspect, the present invention relates to the use of the kit of the eighth aspect of the invention for prognosis and follow-up of the treatment of allergy to proteins in cow's milk with oral immunotherapy with cow's milk.

### BRIEF DESCRIPTION OF THE DRAWINGS

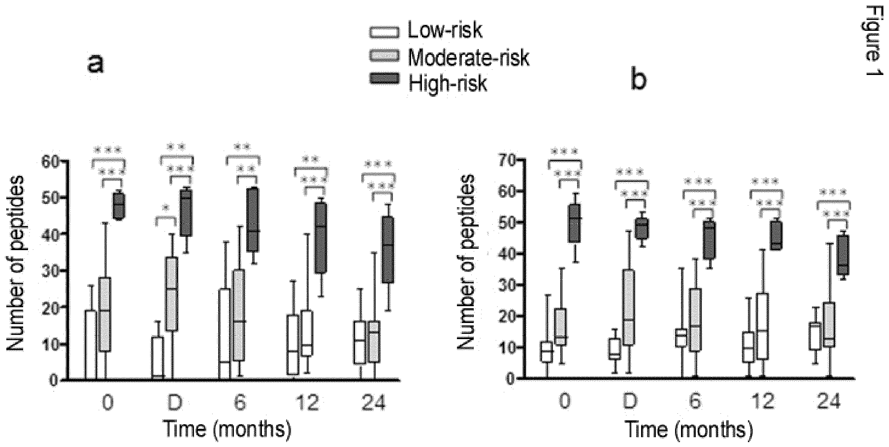

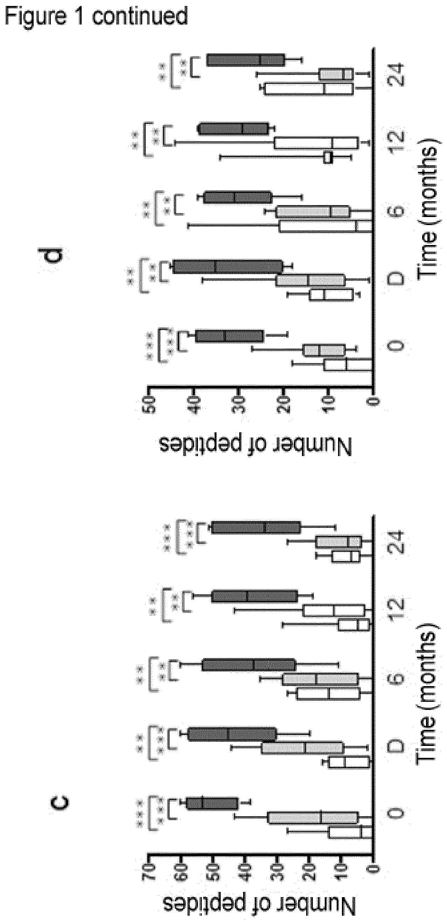

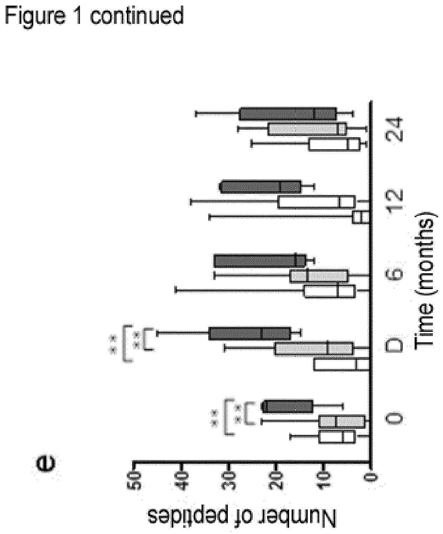

Figure 1 depicts the time course of the number of peptides recognized by patients by means of IgE (0, time before starting OIT treatment; D, time in which tolerance/desensitization is reached; 6, 12 and 24 months of follow-up after desensitization). High-risk patients recognize a greater number of peptides by IgE even before starting OIT treatment, which allows distinguishing high-risk patients from moderate- and low-risk patients. The number of IgE peptides recognized by the different groups of patients decreases with follow-up time which provides information about the minimum duration the OIT must have with cow's milk. a: IgE-alpha-S1-casein; b: IgE-alpha-S2-casein, c: IgE-b-casein, d: IgE-b-lactoglobulin, e: IgE-k-casein.

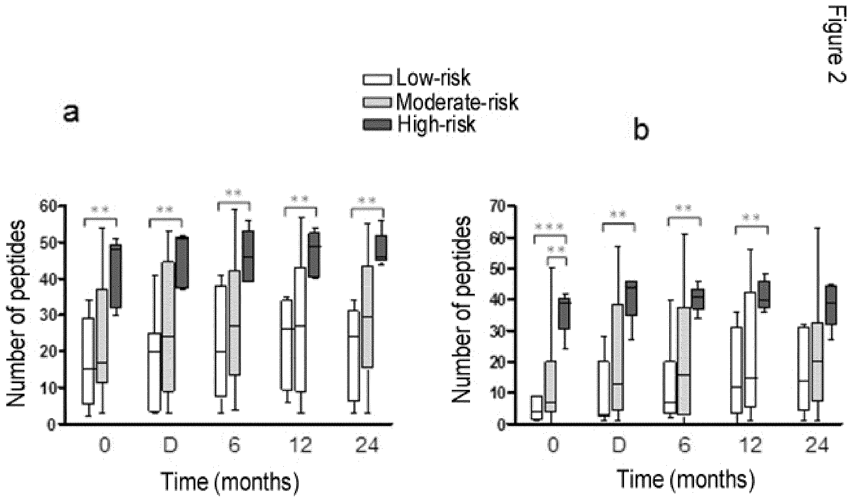

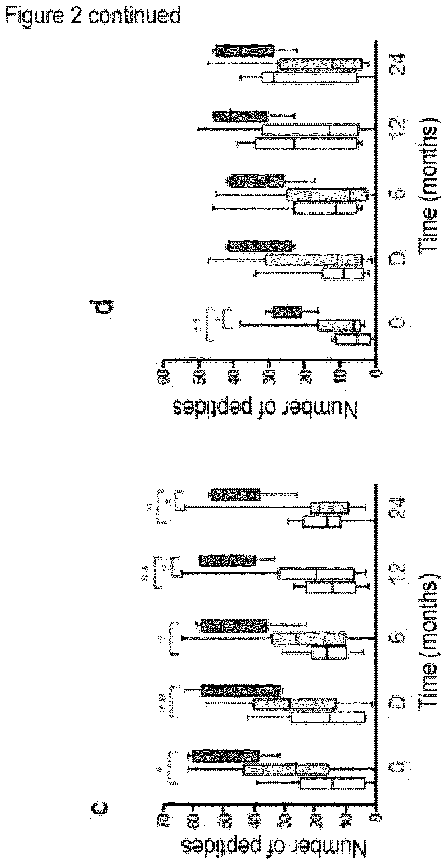

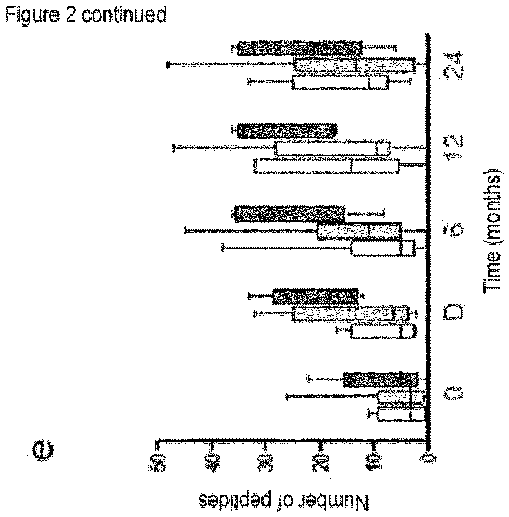

Figure 2 depicts the time course of the number of peptides recognized by patients' IgG4 (0, time before starting OIT treatment; D, time in which tolerance/desensitization is reached; 6, 12 and 24 months of follow-up after desensitization). The number of peptides recognized by the different groups of patients increases during follow-up time which is indicative of the maintenance of tolerance and therefore the efficacy of the OIT treatment with cow's milk. a: IgG4-alpha-S1-casein; b: IgG4-alpha-S2-casein, c: IgG4-b-casein, d: IgG4-b-lactoglobulin, e: IgG4-k-casein.

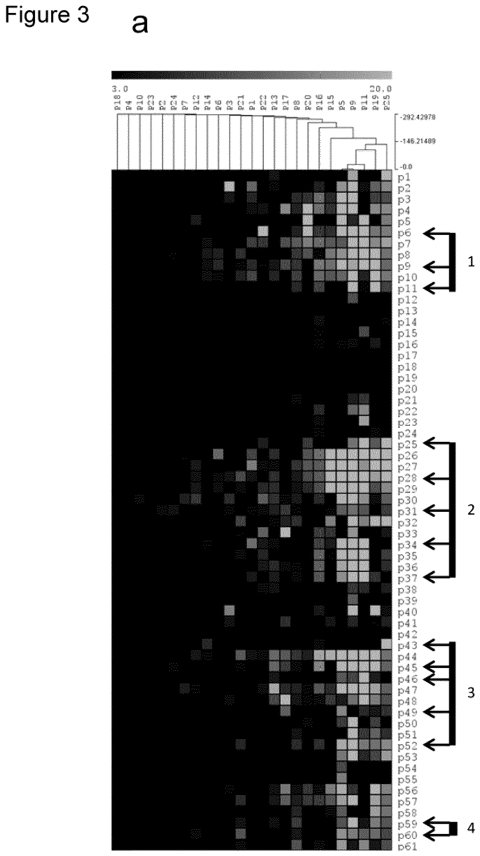

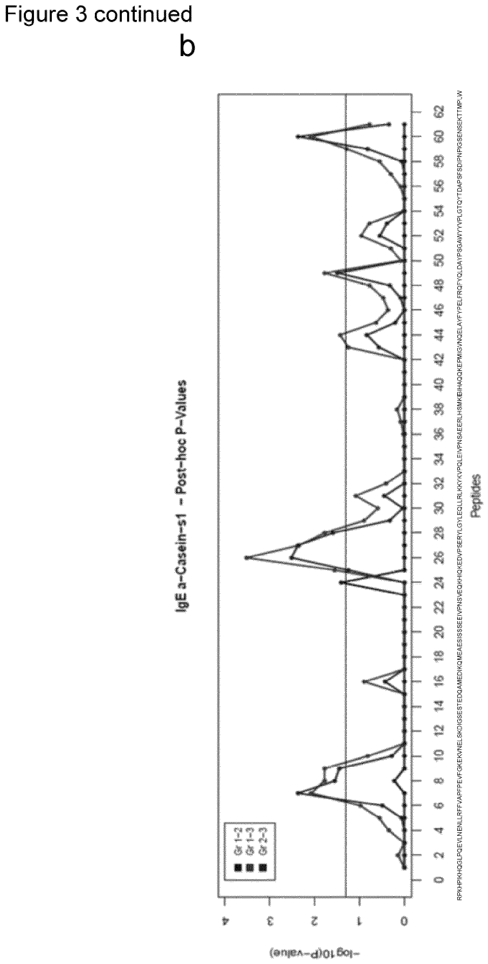

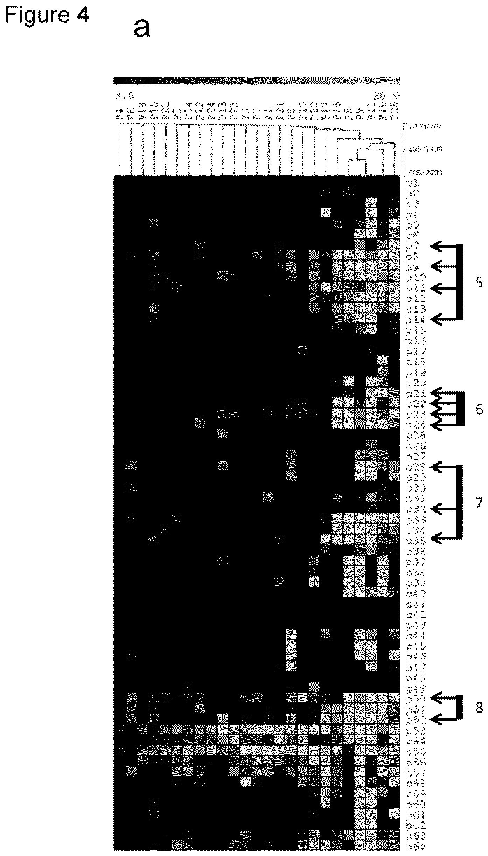

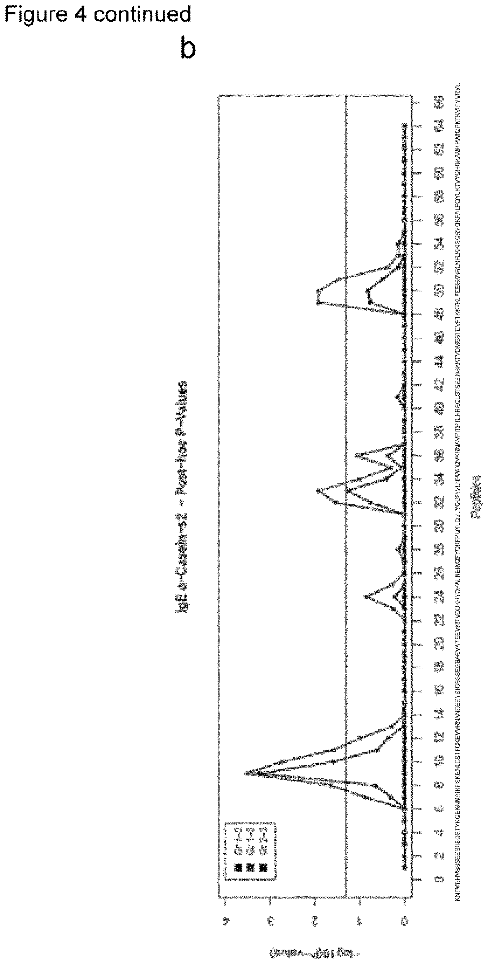

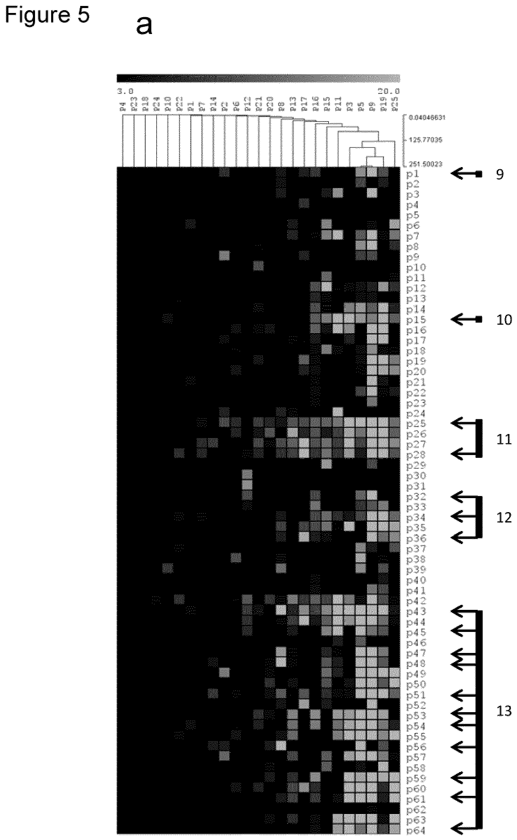

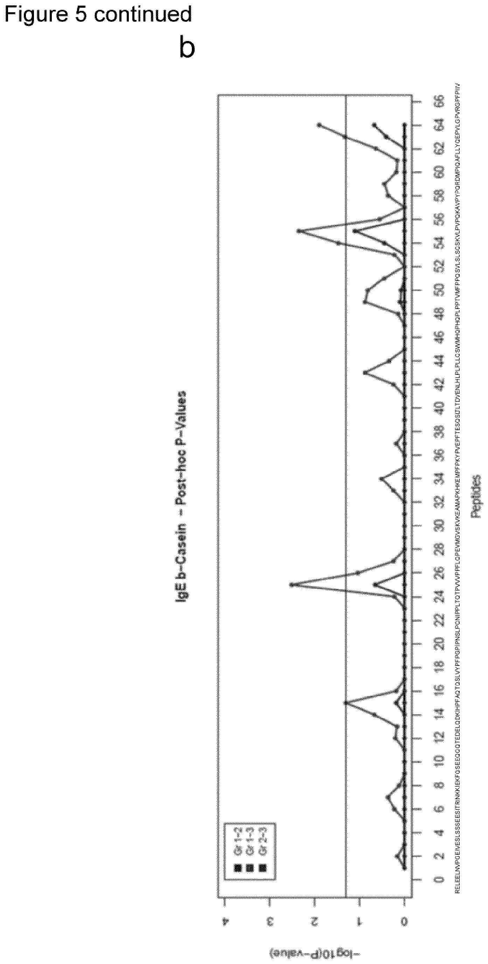

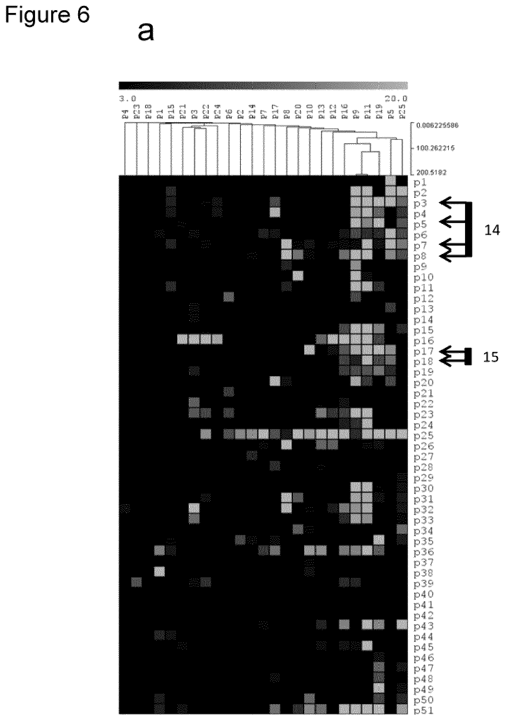

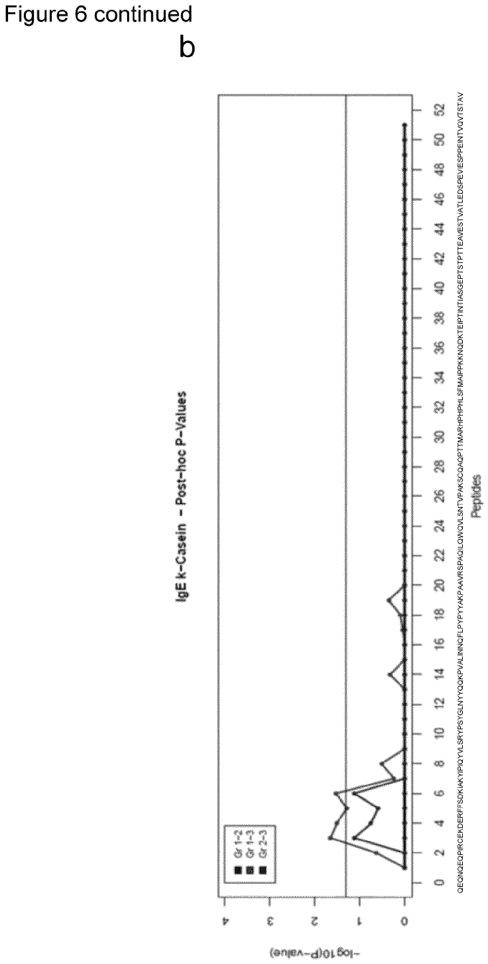

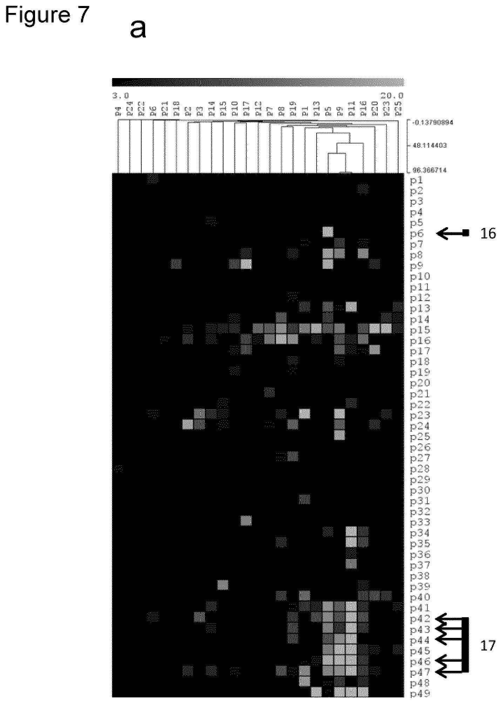

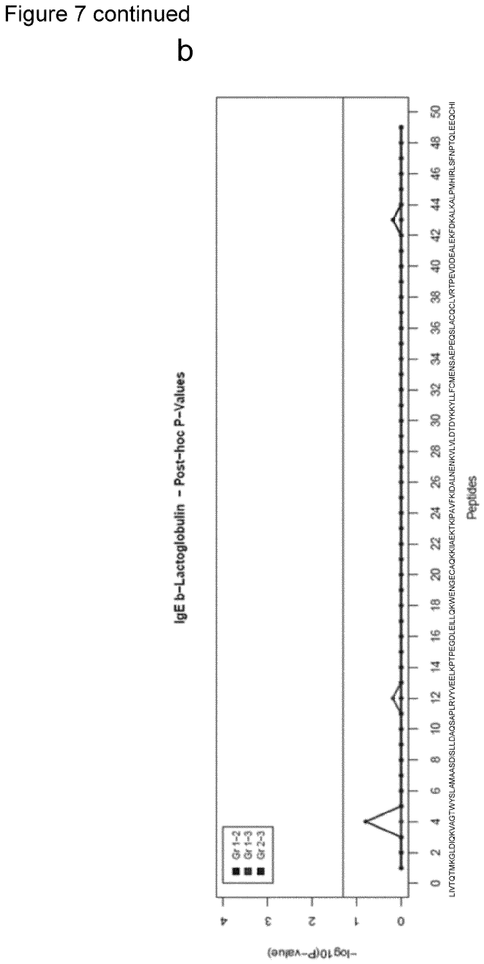

Figures 3 to 7 show the hierarchical cluster analysis, the statistical analysis of the different regions recognized by the different groups of patients (statistical analysis -Gr 1 low-risk patients, Gr 2 moderate-risk patients and Gr 3 high-risk patients-) and the selection of important peptides (key peptide biomarker) (arrows) for patient classification. By means of cluster analysis it can be seen that the immunological recognition patterns by IgE antibodies before starting treatment (time 0) on the high-risk patients are very similar and they are grouped on the right-hand side of the graph (p5, p9, p11, p19 and p25). Those zones or epitopes with significant differences in the recognition pattern of high-risk patients with respect to low- and moderate-risk patients have been located by means of statistical analysis. The best peptides for estimating the progression of OIT treatment have been located by means of machine learning bioinformatics analysis. Fifty-eight peptides (see sequences in Figure 8) of alpha-s1-casein, alpha-s2-casein, beta-casein, kappa-casein and beta-lactoglobulin proteins which are important for estimating the progression of OIT treatment have been selected based on the foregoing,
   Figure 3: alpha-S1-casein (IgE), unweighted,
   Figure 4: alpha-S2-casein (IgE), unweighted,
   Figure 5: b-casein (IgE), unweighted,
   Figure 6: k- casein (IgE), unweighted,
   Figure 7: b-lactoglobulin (IgE), unweighted.
Figure 8 shows the linear correlation between the number of recognized peptides from the 58 peptides shown in Table I and the number of allergic reactions during OIT treatment (r² 0.803) and the treatment time required to achieve tolerance or desensitization (r² 0.601) in different patients.
Figure 9 shows the correlation between the number of peptides from that list recognized by IgE before starting OIT treatment and the number of allergic reactions during treatment of different patients
Figure 10 shows the correlation between the number of peptides from the list (Table 3) recognized by IgE before starting OIT treatment and the treatment time required to achieve tolerance or desensitization in different patients.

### DETAILED DISCLOSURE OF THE INVENTION

### Definitions

In the context of the present invention, a "good prognosis" refers to a low probability of having adverse reactions during desensitization (<7 reactions) and requiring a short time to achieve tolerance to cow's milk (< 8 weeks) and not requiring pre-medication (both antihistamines and corticoids).

In the context of the present invention, a "moderate prognosis" refers to a high number of allergic reactions during desensitization (>7 reactions) or requiring a long time to achieve tolerance to cow's milk (>8 weeks) or requiring antihistamines as pre-medication.

In the context of the present invention, a "poor prognosis" refers to a high number of allergic reactions during desensitization (>7 reactions) and requiring a long time to achieve tolerance to cow's milk (>8 weeks) and requiring antihistamines and corticoids as pre-medication.

In the context of the present invention, tolerance/desensitization is defined as the consumption of 200 ml of cow's milk without having adverse reactions.

In the context of the present invention, pre-medication refers to the administration of antihistamines and/or corticoids before each of the phases of the OIT.

In the context of the present invention, "IgE antibodies against or with specificity for" refers to IgE antibodies recognizing any of the sequences of the 58 epitopes/peptides shown in Table 1, preferably with an intensity threshold equal to or greater than 3 (z-score>3) read in a ScanArray Express fluorescence scanner (PerkinElmer, Waltham, MA, USA) with a 543 nm laser for detecting the signal of IgE-Alexa 546.

In the context of the present invention, derivatives of sequences of the invention that are immunologically active are understood as those fragments of any of sequences SEQ ID No 19-75 that can be epitopes or antigenic determinants to which type E immunoglobulins (IgE) bind, preferably with an intensity threshold equal to or greater than 3 (z-score>3) read in a ScanArray Express fluorescence scanner (PerkinElmer, Waltham, MA, USA) with a 543 nm laser for detecting the signal of IgE-Alexa 546, of patients allergic to cow's milk and which can be determined by means of an immunoassay.

In the context of the present invention, type E immunoglobulins (IgE) are understood as the isotype of type E immunoglobulins, also known as type E plasma antibodies. IgG4 is understood as the isotype of type G4 immunoglobulins, also known as type G4 plasma antibodies.

### DETAILED DISCLOSURE OF THE INVENTION

The immunological recognition pattern (IgE and IgG4 recognition) for proteins in cow's milk (alpha-s1-casein, alpha-s2-casein, beta-casein, kappa-casein and beta-lactoglobulin) in allergic patients during OIT treatment was analyzed by means of peptide microarrays. The times analyzed were:
- Before treatment (0);
- Once tolerance is achieved by means of OIT (D); and
- 6, 12 and 24 months after achieving tolerance (6, 12 and 24, respectively).

An OIT protocol for the administration of cow's milk in two phases was designed, an initial phase consisting of four days with progressive doses each day and another six days administering a single dose twice a week until reaching the full dose of 200 ml of cow's milk. This guideline was modified according to the tolerance to same, occurrence of intercurrent pathologies not related to the study (fever, cold episodes, other infectious processes particularly digestive processes, etc.). All the reactions that appeared during administration of cow's milk both in the hospital and outside the health facility were recorded and quantified.

Patients with persistent allergy to proteins in cow's milk were selected.

The patient inclusion criteria were:
Children over 4 years of age diagnosed with APCW who would not have achieve tolerance spontaneously.

To check the persistence of APCW during the inclusion of patients into the study, skin testing and determination of IgE specific against cow's milk and fractions thereof (beta-lactoglobulin, alpha-lactalbumin and casein) were carried out and a double-blind, placebo-controlled provocation was performed with respect to cow's milk.

Positive skin testing and the presence of IgE specific against cow's milk or any of its fractions and a positive provocation with exposure to the cow's milk were required to reproduce immediate allergy symptoms.

Skin testing was considered positive when it had a size equal to or greater than that produced by histamine and determination of specific IgE was considered positive when it exceeded 0.35 kU/L determined by CAP (Thermo Fisher Scientific).

Children diagnosed with APCW who failed to comply with the previously defined criteria and children with lactose intolerance or reactions not mediated by IgE were excluded.

A total of 47 patients were included, from that 12 were excluded as they showed an exclusion criterion. A total of 35 children complied with the inclusion criteria and they were all offered OIT treatment with cow's milk. From the 35 children, a total of 24 accepted the treatment and another 7 children rejected the treatment but agreed to remain in the study as a control group.

In terms of the efficacy of the treatment, efficacy was considered reaching the amount of 200 ml of cow's milk a day without adverse reactions.

In terms of the safety of the guideline of OIT with cow's milk, the number of allergic reactions presented by each patient during the procedure, the need for protection with anti-allergy medication (corticoids and antihistamines) and the duration of the guideline were considered.

Twenty-four included patients reached the dose of 200 ml, therefore the procedure was effective in all cases.

The patients were classified according to duration longer or less than 8 weeks (median procedure duration with all patients), number of reactions of more than 7 (median group reactions) and whether or not they need anti-allergy medication (antihistamines and/or corticoids) for administration of the doses (Yes, No).

According to these criteria, the patients were classified into three groups
- Low risk (less than 8 weeks, less than 7 reactions and do not need medication). Seven patients showed this profile,
- Moderate risk (more than 8 weeks and/or more than 7 reactions and/or pre-medication with antihistamines). Twelve patients showed this profile,
- High risk (more than 8 weeks and more than 7 reactions and complete pre-medication (antihistamines and corticoids). Five patients showed this profile.

Once the test was performed, the authors of the present invention correlated the number of peptides recognized by IgE antibodies in the sera of the patients for the different proteins before starting OIT treatment (time 0) with the response after said treatment. The low-risk patients recognized a few peptides, the high-risk patients recognized a lot of peptides and the moderate-risk patients recognized an intermediate number of peptides. Fifty-eight epitopes/peptides of the five proteins responsible for APCW were identified from this test, providing information for prognosis of the response to OIT against cow's milk (Table 1 and Figures 3 to 7). Furthermore, the time course in the recognition of these 58 epitopes/peptides by IgE (and optionally IgG4) allows determining the duration of the OIT (increased IgG4 recognition and decreased IgE recognition), and indicating the patient's immunological tolerance/desensitization status (the patient tolerates food requiring uninterrupted, daily exposure to the allergen) or permanent tolerance (the patients tolerates food and suspension thereof for a time period, the state of tolerance is unchanged), and therefore the overall efficacy of the OIT with cow's milk.

By means of bioinformatics analysis, a subgroup of 27 epitopes/peptides having a high prognosis value for prognosis of the number of reactions during OIT (Table 3 and Figure 9) and the treatment time required to achieve tolerance (Table 4 and Figure 10) was located from among the 58 epitopes/peptides described above.

Table 3 shows reference values based on 16 peptides recognized by IgE antibodies in the sera of 24 patients for estimating the number of reactions during oral immunotherapy (OIT).

Table 4 shows reference values based on 16 peptides recognized by IgE antibodies in the sera of 24 patients for estimating the treatment time required to achieve tolerance or desensitization during OIT.

To correlate the number of peptide sequences recognized by IgE antibodies in the sera of the subjects before starting OIT treatment (time 0) with the reference values established based on those same peptide sequences which correlate the number of peptides recognized by IgE antibodies in the sera of the patients with the response after OIT treatment, algorithms or mathematical formulas, such as for example, regression lines such as those included in Table 3 and 4, can be used.

Therefore, by way of example, the reference values obtained from the 24 patients previously analyzed could be used for estimating the number of reactions during OIT and the treatment time required to achieve tolerance or desensitization. Therefore, for estimating the number of reactions, the relationship between the number of peptides in Table 2 recognized by each of the 24 reference patients and the number of reactions each of them experienced during OIT could be modeled by means of a linear fit or linear regression model (Figure 9), thereby establishing an equation which allows estimating in new patients the number of reactions they will experience during OIT based on the number of recognized peptides before starting treatment.

Additionally and for estimating the treatment time required to achieve tolerance or desensitization during OIT, the relationship between the number of peptides in Table 3 recognized by each of the 24 reference patients and the time required by each of them to achieve tolerance could be modeled by means of a linear fit or linear regression model (Figure 10) and thereby establishing an equation which allows estimating in new patients the time required to achieve tolerance during OIT based on the number of recognized peptides before starting treatment.

Therefore, as can be seen in Figures 8-10, by analyzing the IgE recognition pattern of the immunologically active sequences of the 58 epitopes/peptides shown in Table 1, as well as of subsets of said sequences such as the 16 peptides shown in Table 3 or the 16 peptides shown in Table 4, a series of reference values (see Figures 8-10) have been established which allows, depending on the number of peptides recognized by IgE in a sample isolated from a patient before starting OIT treatment, estimating the number of adverse reactions and the time required to achieve desensitization as well as whether or not pre-medication is needed. Furthermore, it allows classifying the patients as low-risk, moderate-risk or high-risk patients depending on the number of peptides recognized by IgE before starting the OIT.

**Table 1**

| ID | Protein | Position | Sequence |
|---|---|---|---|
| 1 | alpha-s1-casein | 6 | SEQ ID NO: 18 |
| 2 | alpha-s 1-casein | 9 | SEQ ID NO: 19 |
| 3 | alpha-s 1-casein | 11 | SEQ ID NO: 20 |
| 4 | alpha-s 1-casein | 25 | SEQ ID NO: 21 |
| 5 | alpha-s 1-casein | 28 | SEQ ID NO: 22 |
| 6 | alpha-s 1-casein | 31 | SEQ ID NO: 23 |
| 7 | alpha-s 1-casein | 34 | SEQ ID NO: 24 |
| 8 | alpha-s 1-casein | 37 | SEQ ID NO: 25 |
| 9 | alpha-s 1-casein | 43 | SEQ ID NO: 26 |
| 10 | alpha-s 1-casein | 45 | SEQ ID NO: 27 |
| 11 | alpha-s 1-casein | 46 | SEQ ID NO: 28 |
| 12 | alpha-s1-casein | 49 | SEQ ID NO: 29 |
| 13 | alpha-s1-casein | 52 | SEQ ID NO: 30 |
| 14 | alpha-s1-casein | 59 | SEQ ID NO: 31 |
| 15 | alpha-s1-casein | 60 | SEQ ID NO: 32 |
| 16 | alpha-s2-casein | 7 | SEQ ID NO: 33 |
| 17 | alpha-s2-casein | 9 | SEQ ID NO: 34 |
| 18 | alpha-s2-casein | 11 | SEQ ID NO: 35 |
| 19 | alpha-s2-casein | 14 | SEQ ID NO: 36 |
| 20 | alpha-s2-casein | 21 | SEQ ID NO: 37 |
| 21 | alpha-s2-casein | 22 | SEQ ID NO: 38 |
| 22 | alpha-s2-casein | 23 | SEQ ID NO: 39 |
| 23 | alpha-s2-casein | 24 | SEQ ID NO: 40 |
| 24 | alpha-s2-casein | 28 | SEQ ID NO: 41 |
| 25 | alpha-s2-casein | 32 | SEQ ID NO: 42 |
| 26 | alpha-s2-casein | 35 | SEQ ID NO: 43 |
| 27 | alpha-s2-casein | 50 | SEQ ID NO: 44 |
| 28 | alpha-s2-casein | 52 | SEQ ID NO: 45 |
| 29 | beta-casein | 1 | SEQ ID NO: 46 |
| 30 | beta-casein | 15 | SEQ ID NO: 47 |
| 31 | beta-casein | 25 | SEQ ID NO: 48 |
| 32 | beta-casein | 28 | SEQ ID NO: 49 |
| 33 | beta-casein | 32 | SEQ ID NO: 50 |
| 34 | beta-casein | 34 | SEQ ID NO: 51 |
| 35 | beta-casein | 36 | SEQ ID NO: 52 |
| 36 | beta-casein | 43 | SEQ ID NO: 53 |
| 37 | beta-casein | 45 | SEQ ID NO: 54 |
| 38 | beta-casein | 47 | SEQ ID NO: 55 |
| 39 | beta-casein | 48 | SEQ ID NO: 56 |
| 40 | beta-casein | 51 | SEQ ID NO: 57 |
| 41 | beta-casein | 53 | SEQ ID NO: 58 |
| 42 | beta-casein | 54 | SEQ ID NO: 59 |
| 43 | beta-casein | 56 | SEQ ID NO: 60 |
| 44 | beta-casein | 59 | SEQ ID NO: 61 |
| 45 | beta-casein | 61 | SEQ ID NO: 62 |
| 46 | beta-casein | 64 | SEQ ID NO: 63 |
| 47 | kappa-casein | 3 | SEQ ID NO: 64 |
| 48 | kappa-casein | 5 | SEQ ID NO: 65 |
| 49 | kappa-casein | 7 | SEQ ID NO: 66 |
| 50 | kappa-casein | 8 | SEQ ID NO: 67 |
| 51 | kappa-casein | 17 | SEQ ID NO: 68 |
| 52 | kappa-casein | 18 | SEQ ID NO: 69 |
| 53 | beta-lactoglobulin | 6 | SEQ ID NO: 70 |
| 54 | beta-lactoglobulin | 42 | SEQ ID NO: 71 |
| 55 | beta-lactoglobulin | 43 | SEQ ID NO: 72 |
| 56 | beta-lactoglobulin | 44 | SEQ ID NO: 73 |
| 57 | beta-lactoglobulin | 46 | SEQ ID NO: 74 |
| 58 | beta-lactoglobulin | 47 | SEQ ID NO: 75 |

Table 1 shows the 58 epitopes/peptides of the five proteins responsible for APCW.

Table 2 shows the 17 sequences (see Figures 3-7) included in the 58 epitopes/peptides of the five proteins responsible for APCW.

**Table 2**

| ID | Sequence |
|---|---|
| 1 | LNENLLRFFVAPFPEVFGKEKVNELSKDIGSESTE |
| 2 | |
| 3 | IHAQQKEPMIGVNQELAYFYPELFRQFYQLDAYPSGAWYYVPLGTQ |
| 4 | DAPSFSDIPNPIGSENSEKTTMP |
| 5 | TYKQEKNMAINPSKENLCSTFCKEVVRNANEEEYSIGSSSE |
| 6 | SAEVATEEVKITVDDKHYQKALNEINQFY |
| 7 | LNEINQFYQKFPQYLQYLYQGPIVLNPWDQVKRNAVPITPT |
| 8 | TKKTKLTEEEKNRLNFLKKISQRYQK |
| 9 | RELEELNVPGEIVESLSSSE |
| 10 | DELQDKIHPFAQTQSLVYPF |
| 11 | NIPPLTQTPWVPPFLQPEVMGVSKVKEA |
| 12 | GVSKVKEAMAPKHKEMPFPKYPVEPFTESQSL |
| 13 | |
| 14 | QPIRCEKDERFFSDKIAKYIPIQYVLSRYPSYGLN |
| 15 | ALINNQFLPYPYYAKPAAVRSPA |
| 16 | AGTWYSLAMAASDISLLDAQ |
| 17 | RTPEVDDEALEKFDKALKALPMHIRLSFNPTQLEE |

The following examples are merely intended for illustrating the invention and in no case for limiting same.

### Example 1:

Using a SpotArray 72 microarrays printing robot (PerkinElmer, Waltham, MA, USA) microarrays were printed on NSB27 NHS glass functionalized with N-hydroxyl succinimidyl (NanoSurface Biosciences-POSTECH, Seoul, Korea) containing 27 peptides with ID: 2 (α-s1-casein p9), 4 (α-s1-casein p25), 8 (α-s1-casein p37), 10 (α-s1-casein p45), 11 (α-s1-casein p46), 14 (α-s1-casein p59), 19 (alpha-s2-casein p14), 20 (alpha-s2-casein p21), 21 (alpha-s2-casein p22), 22 (alpha-s2-casein p23), 23 (alpha-s2-casein p24), 27 (alpha-s2-casein p50), 34 (beta-casein p34), 36 (beta-casein p43), 38 (beta-casein p47), 39 (beta-casein p48), 41 (beta-casein p53), 42 (beta-casein p54), 45 (beta-casein p61), 50 (kappa-casein p8), 51 (kappa-casein p17), 52 (kappa-casein p18), 53 (beta-lactoglobulin p6), 54 (beta-lactoglobulin p42), 55 (beta-lactoglobulin p43), 56 (beta-lactoglobulin p44) and 58 (beta-lactoglobulin p47); see Table 3 and 4 and Figure 9 and 10. For printing, the peptides are dissolved in a 1:1 phosphate saline buffer (PBS) solution and printing buffer (PPB) (protein printing buffer, Arrayit Corporation, Sunnyvale, CA, USA). Once the microarrays were printed, non-specific bindings were blocked for 1 hour at room temperature with a 1:1 Blocklt™ blocking solution (Arrayit Corporation, Sunnyvale, CA, USA) and PBS containing 0.1% Tween 20 and 2% Bovine Serum Albumin (PBS-T-BSA).

The sera obtained from 3 patients were incubated on the surface of the microarrays for 16 hours at 4°C and under continuous stirring. The sample was removed from the microarrays and washed twice with a PBS-T-BSA solution. They were then incubated with a solution containing anti-human IgE fluorescent antibodies (G7-26, BD-PharMingen, San Diego, CA, USA) labeled with Alexa 546 and anti-human IgG4 fluorescent antibodies (G14-4, BD-PharMingen, San Diego, CA, USA) labeled with Alexa 647, diluted at 1/1000 in PBS-T-BSA. The solution containing antibodies was removed and washed twice with a PBS-T-BSA solution.

To detect the presence in the serum of the patients of IgE and IgG4 antibodies against the different peptides of the allergen proteins in cow's milk, the microarrays were read in a ScanArray Express fluorescence scanner (PerkinElmer, Waltham, MA, USA) with two lasers (a 543 nm laser for detecting the signal of IgE-Alexa 546 and a 633 nm laser for detecting the signal of IgG4-Alexa 647). The results were quantified and standardized for transforming the fluorescence signal into a numerical parameter (arbitrary fluorescence units or z-score). The number of peptides with a fluorescence signal greater than 3 (z-score>3) was calculated. The first patient recognized 2 peptides, the second patient recognized 8 peptides and the third patient recognized 15 peptides.

The patient with a peptide recognition of 2 has a good prognosis (low probability of having adverse reactions during desensitization - an estimate of 3 allergic reactions, see Table 3 and Figure 9- and requiring a short time to achieve tolerance to cow's milk - an estimate of 7 weeks, see Table 4 and Figure 10-).

Patients with a peptide recognition of 8 has an intermediate prognosis (moderate risk of having adverse reactions during desensitization - an estimate of 10 allergic reactions, see Table 3 and Figure 9- and requiring an intermediate time to achieve tolerance to cow's milk - an estimate of 11 weeks, see Table 4 and Figure 10).

Patients with a peptide recognition of 15 has a poor prognosis (high probability of having adverse reactions during desensitization) - an estimate 19 allergic reactions, see Table 2 and Figure 9- and requiring a long time to achieve tolerance to cow's milk - an estimate of 16 weeks. Additionally, these patients will require pre-medication and treatment with antihistamines and corticoids before each of the phases of the OIT (see Table 4 and Figure 10).

**Table 3**

| ID | Protein | Sequence |
|---|---|---|
| 2 | α-s1-casein p9 | SEQ ID NO:19 |
| 4 | α-s1-casein p25 | SEQ ID NO: 21 |
| 8 | α-s1-casein p37 | SEQ ID NO: 25 |
| 10 | α-s1-casein p45 | SEQ ID NO: 27 |
| 19 | alpha-s2-casein p14 | SEQ ID NO: 36 |
| 20 | alpha-s2-casein p21 | SEQ ID NO: 37 |
| 21 | alpha-s2-casein p22 | SEQ ID NO: 38 |
| 34 | beta-casein p34 | SEQ ID NO: 51 |
| 36 | beta-casein p43 | SEQ ID NO: 53 |
| 41 | beta-casein p53 | SEQ ID NO: 58 |
| 42 | beta-casein p54 | SEQ ID NO: 59 |
| 45 | beta-casein p61 | SEQ ID NO: 62 |
| 52 | kappa-casein p18 | SEQ ID NO: 69 |
| 54 | beta-lactoglobulin p42 | SEQ ID NO: 71 |
| 55 | beta-lactoglobulin p43 | SEQ ID NO: 72 |
| 56 | beta-lactoglobulin p44 | SEQ ID NO: 73 |

Table 3: peptides that best estimate the number of reactions during OIT Formula: Estimated reactions =1.1733 No. of peptides + 1.0362
No. of peptides: 2, Estimated reactions: 3
No. of peptides: 8, Estimated reactions: 10
No. of peptides: 15, Estimated reactions: 19

**Table 4**

| ID | Peptide | Sequence |
|---|---|---|
| 11 | α-s1-casein p46 | SEQ ID NO: 28 |
| 14 | α-s1-casein p59 | SEQ ID NO: 31 |
| 19 | alpha-s2-casein p14 | SEQ ID NO: 36 |
| 20 | alpha-s2-casein p21 | SEQ ID NO: 37 |
| 21 | alpha-s2-casein p22 | SEQ ID NO: 38 |
| 22 | alpha-s2-casein p23 | SEQ ID NO: 39 |
| 23 | alpha-s2-casein p24 | SEQ ID NO: 40 |
| 27 | alpha-s2-casein p50 | SEQ ID NO: 44 |
| 36 | beta-casein p43 | SEQ ID NO: 53 |
| 38 | beta-casein p47 | SEQ ID NO: 55 |
| 39 | beta-casein p48 | SEQ ID NO: 56 |
| 50 | kappa-casein p8 | SEQ ID NO: 67 |
| 51 | kappa-casein p17 | SEQ ID NO: 68 |
| 53 | beta-lactoglobulin p6 | SEQ ID NO: 70 |
| 55 | beta-lactoglobulin p43 | SEQ ID NO: 72 |
| 58 | beta-lactoglobulin p47 | SEQ ID NO: 75 |

Table 4: peptides that best estimate the treatment time required to achieve tolerance or desensitization during OIT.

Formula: Estimated treatment time (weeks) = (0.7318 * No. of peptides) + 5.1419
No. of peptides: 2, estimated treatment time (weeks): 7
No. peptides: 8, estimated treatment time (weeks): 11
No. peptides: 15, estimated treatment time (weeks): 16

## Claims

1. A method for the prognosis of oral immunotherapy (OIT) against proteins in cow's milk in a human subject unable to tolerate cow's milk, comprising determining the presence or absence of IgE antibodies in an isolated biological sample from the subject taken before starting OIT treatment (time 0), such as blood, serum, plasma, cerebrospinal fluid, peritoneal fluid, faces or urine, capable of recognizing peptides identified with any of the following sequences:
a. At least one sequence selected from the group consisting of sequences SEQ ID NO: 18-32; and/or
b. At least one sequence selected from the group consisting of sequences SEQ ID NO: 33-45; and/or
c. At least one sequence selected from the group consisting of sequences SEQ ID NO: 46-63; and/or
d. At least one sequence selected from the group consisting of sequences SEQ ID NO: 64-69; and/or
e. At least one sequence selected from the group consisting of sequences SEQ ID NO: 70-75;
wherein the prognosis of oral immunotherapy (OIT) against proteins in cow's milk is established by correlating the number of peptide sequences recognized by IgE antibodies in the sample from the subject before starting OIT treatment (time 0) with reference values established based on those same peptide sequences which correlate the number of peptides recognized by IgE antibodies in the samples from the patients with the response after OIT treatment.

2. The method according to claim 1, where said method is a method for the prognosis of the number of reactions during oral immunotherapy (OIT) and the determination of the presence or absence of IgE antibodies in the biological sample, wherein said method is performed by determining the presence or absence of IgE antibodies capable of recognizing peptides identified with any of the following sequences:
a. sequences SEQ ID NO: 19, 21, 25 and 27; and
b. sequences SEQ ID NO: 36, 37 and 38; and
c. sequences SEQ ID NO: 51, 53, 58, 59 and 62; and
d. sequence SEQ ID NO: 69; and
e. sequences SEQ ID NO: 71 and 72.

3. The method according to claim 1, wherein the method is a method for the prognosis of an estimate of the treatment time required to achieve tolerance or desensitization during OIT and the determination of the presence or absence of IgE antibodies in the biological sample, wherein the method is performed by determining the presence or absence of IgE antibodies capable of recognizing peptides identified with any of the following sequences:
a. sequences SEQ ID NO: 28 and 31; and
b. sequences SEQ ID NO: 36-40 and 44; and
c. sequences SEQ ID NO: 53, 55 and 56; and
d. sequences SEQ ID NO: 67 and 68; and
e. sequences SEQ ID NO: 70, 72 and 75.

4. The method according to claim 1, where the determination of the presence or absence of IgE antibodies in the biological sample is performed by determining the presence or absence of IgE antibodies capable of recognizing peptides identified with the following sequences: SEQ ID NO: 18-75, wherein:
a. a peptide recognition of less than 15% of said sequences is indicative of a good prognosis, wherein good prognosis refers to a low probability of having adverse reactions during desensitization (<7 reactions) and requiring a short time to achieve tolerance to cow's milk (< 8 weeks) and not requiring pre-medication;a peptide recognition of between 15%-75% of said sequences is indicative of a moderate prognosis, wherein moderate prognosis refers to a high number of allergic reactions during desensitization (>7 reactions) or requiring a long time to achieve tolerance to cow's milk (>8 weeks) or requiring antihistamines as pre-medication; and
b. a peptide recognition of more than 75% of said sequences is indicative of a poor prognosis, wherein poor prognosis refers to a high number of allergic reactions during desensitization (>7 reactions) and requiring a long time to achieve tolerance to cow's milk (>8 weeks) and requiring antihistamines and corticoids as premedication.

5. The method according to any of the preceding claims, wherein peptide recognition is determined by means of an immunoassay.

6. The method according to any of claims 1 to 4, wherein peptide recognition is determined by means of peptide microarrays.

7. Use of a peptide microarray suitable for carrying out the method of any of claims 1-4 comprising the combinations of peptides as defined in any of claims 1-4, for the prognosis of oral immunotherapy (OIT) against proteins in cow's milk in a human subject.

8. *In vitro* use of a kit comprising peptides as capture biomolecules, wherein said peptides are selected from the list consisting of any one of SEQ ID NO: 18-75, for the prognosis of the number of reactions during oral immunotherapy (OIT) against proteins in cow's milk and/or an estimate of the treatment time required to achieve tolerance or desensitization during OIT and/or the need to apply pre-medication during OIT.

9. *In vitro* use of a kit comprising:
a. Capture biomolecules capable of recognizing IgE selected from the list consisting of any one of SEQ ID NO: 18-75 or consisting of the specific subsets of peptides as defined in any of claims 3 or 4; and
b. A second recognition molecule (detection biomolecule) capable of recognizing the target analyte or the capture biomolecule optionally bound to a tag molecule;
for the prognosis of the number of reactions during oral immunotherapy (OIT) against proteins in cow's milk and/or an estimate of the treatment time required to achieve tolerance or desensitization during OIT and/or the need to apply pre-medication during OIT

10. Use of a kit comprising:
a. Capture biomolecules capable of recognizing IgE selected from the list consisting of any one of SEQ ID NO: 18-75 or consisting of the specific subsets of peptides as defined in any of claims 3 or 4;
b. A support where the capture biomolecules of step a) are immobilized; and
c. A second recognition molecule (detection biomolecule) capable of recognizing the target analyte or the capture biomolecule optionally bound to a tag molecule;
for the prognosis of the number of reactions during oral immunotherapy (OIT) against proteins in cow's milk and/or an estimate of the treatment time required to achieve tolerance or desensitization during OIT and/or the need to apply pre-medication during OIT

## Patentansprüche

1. Verfahren zur Prognose einer oralen Immuntherapie (OIT) gegen Proteine in Kuhmilch in einem menschlichen Individuum, welches nicht in der Lage ist Kuhmilch zu tolerieren, umfassend das Bestimmen der Anwesenheit oder Abwesenheit von IgE-Antikörpern in einer isolierten biologischen Probe des Individuums, welche vor Beginn der OIT-Behandlung (Zeit 0) entnommen wurde, wie Blut, Serum, Plasma, Gehirn-Rückenmark-Flüssigkeit, Peritonealflüssigkeit, Fäkalien oder Urin, welches in der Lage ist Peptide zu erkennen, welche mit einer der folgenden Sequenzen identifiziert werden:
a. mindestens einer Sequenz ausgewählt aus der Gruppe bestehend aus Sequenzen SEQ ID NO: 18-32; und/oder
b. mindestens einer Sequenz ausgewählt aus der Gruppe bestehend aus Sequenzen SEQ ID NO: 33-45; und/oder
c. mindestens einer Sequenz ausgewählt aus der Gruppe bestehend aus Sequenzen SEQ ID NO: 46-63; und/oder
d. mindestens einer Sequenz ausgewählt aus der Gruppe bestehend aus Sequenzen SEQ ID NO: 64-69; und/oder
e. mindestens einer Sequenz ausgewählt aus der Gruppe bestehend aus Sequenzen SEQ ID NO: 70-75;
wobei die Prognose einer oralen Immuntherapie (OIT) gegen Proteine in Kuhmilch durch die Korrelation der Anzahl von Peptidsequenzen, welche von den IgE-Antikörpern in der Probe des Individuums vor Beginn der OIT-Behandlung (Zeit 0) erkannt werden, mit Referenzwerten festgestellt wird, welche basierend auf den gleichen Peptidsequenzen, welche mit der Anzahl von Peptiden korrelieren, welche von den IgE-Antikörpern in den Proben aus Patienten mit Antwort auf die OIT-Behandlung erkannt werden, festgestellt werden.

2. Verfahren nach Anspruch 1, wobei das genannte Verfahren ein Verfahren zur Prognose der Anzahl von Reaktionen während der oralen Immuntherapie (OIT) und zur Bestimmung der Anwesenheit oder Abwesenheit von IgE-Antikörpern in der biologischen Probe ist, wobei das genannte Verfahren durch das Bestimmen der Anwesenheit oder Abwesenheit von IgE-Antikörpern durchgeführt wird, welche in der Lage sind Peptide zu erkennen, welche mit einer der folgenden Sequenzen identifiziert werden:
a. Sequenzen SEQ ID NO: 19, 21, 25 und 27; und
b. Sequenzen SEQ ID NO: 36, 37 und 38; und
c. Sequenzen SEQ ID NO: 51, 53, 58, 59 und 62; und
d. Sequenz SEQ ID NO: 69; und
e. Sequenzen SEQ ID NO: 71 und 72.

3. Verfahren nach Anspruch 1, wobei das Verfahren ein Verfahren zur Prognose einer Schätzung der Behandlungszeit, welche benötigt wird, um Toleranz oder Desensibilisierung während der OIT zu erreichen, und zur Bestimmung der Anwesenheit oder Abwesenheit von IgE-Antikörpern in der biologischen Probe ist, wobei das Verfahren durch das Bestimmen der Anwesenheit oder Abwesenheit von IgE-Antikörpern durchgeführt wird, welche in der Lage sind Peptide zu erkennen, welche mit einer der folgenden Sequenzen identifiziert werden:
a. Sequenzen SEQ ID NO: 28 und 31; und
b. Sequenzen SEQ ID NO: 36-40 und 44; und
c. Sequenzen SEQ ID NO: 53, 55 und 56; und
d. Sequenzen SEQ ID NO: 67 und 68; und
e. Sequenzen SEQ ID NO: 70, 72 und 75.

4. Verfahren nach Anspruch 1, wobei die Bestimmung der Anwesenheit oder Abwesenheit von IgE-Antikörpern in der biologischen Probe durch das Bestimmen der Anwesenheit oder Abwesenheit von IgE-Antikörpern durchgeführt wird, welche in der Lage sind Peptide zu erkennen, welche mit den folgenden Sequenzen: SEQ ID NO: 18-75 identifiziert werden, wobei:
a. eine Peptiderkennung von weniger als 15% der genannten Sequenzen auf eine gute Prognose schließen lässt, wobei sich eine gute Prognose auf eine niedrige Wahrscheinlichkeit des Aufweisens von unerwünschten Reaktionen während der Desensibilisierung (<7 Reaktionen) und das Benötigen einer kurzen Zeit (< 8 Wochen), um Toleranz gegen Kuhmilch zu erreichen, und das nicht Benötigen von Prämedikation bezieht; eine Peptiderkennung von zwischen 15%-75% der genannten Sequenzen auf eine moderate Prognose schließen lässt, wobei sich eine moderate Prognose auf eine hohe Anzahl von allergischen Reaktionen während der Desensibilisierung (>7 Reaktionen) oder das Benötigen einer langen Zeit (>8 Wochen), um Toleranz gegen Kuhmilch zu erreichen, oder das Benötigen von Antihistaminika als Prämedikation bezieht; und
b. eine Peptiderkennung von mehr als 75% der genannten Sequenzen auf eine schlechte Prognose schließen lässt, wobei sich eine schlechte Prognose auf eine hohe Anzahl von allergischen Reaktionen während der Desensibilisierung (>7 Reaktionen) und das Benötigen einer langen Zeit (>8 Wochen), um Toleranz gegen Kuhmilch zu erreichen, und das Benötigen von Antihistaminika und Kortikoiden als Prämedikation bezieht.

5. Verfahren nach einem der vorhergehenden Ansprüche, wobei die Peptiderkennung mittels eines Immuntests bestimmt wird.

6. Verfahren nach einem der Ansprüche 1 bis 4, wobei die Peptiderkennung mittels Peptidmikroarrays bestimmt wird.

7. Verwendung eines Peptidmikroarrays, welches dafür geeignet ist, das Verfahren nach einem der Ansprüche 1-4 durchzuführen, umfassend die in einem der Ansprüche 1-4 definierten Kombinationen von Peptiden, zur Prognose einer oralen Immuntherapie (OIT) gegen Proteine in Kuhmilch in einem menschlichen Individuum.

8. *In*-vitro-Verwendung eines Kits umfassend Peptide als Fänger-Biomoleküle, wobei die genannten Peptide aus der Liste bestehend aus einer der SEQ ID NO: 18-75 ausgewählt werden, zur Prognose der Anzahl von Reaktionen während der oralen Immuntherapie (OIT) gegen Proteine in Kuhmilch und/oder einer Schätzung der Behandlungszeit, welche benötigt wird, um Toleranz oder Desensibilisierung während der OIT zu erreichen, und/oder des Bedürfnisses Prämedikation während der OIT anzuwenden.

9. *In-vitro*-Verwendung eines Kits umfassend:
a. Fänger-Biomoleküle, welche in der Lage sind, IgE ausgewählt aus der Liste bestehend aus einer der SEQ ID NO: 18-75 oder bestehend aus den spezifischen Teilsätzen von Peptiden, wie in einem der Ansprüche 3 oder 4 definiert werden, zu erkennen; und
b. ein zweites Erkennungsmolekül (*detection biomolecule*)*,* welches in der Lage ist, den Zielanalyten oder das Fänger-Biomolekül, welches wahlweise an einem Markierungsmolekül gebunden ist, zu erkennen;
zur Prognose der Anzahl von Reaktionen während der oralen Immuntherapie (OIT) gegen Proteine in Kuhmilch und/oder einer Schätzung der Behandlungszeit, welche benötigt wird, um Toleranz oder Desensibilisierung während der OIT zu erreichen, und/oder des Bedürfnisses Prämedikation während der OIT anzuwenden.

10. Verwendung eines Kits umfassend:
a. Fänger-Biomoleküle, welche in der Lage sind, IgE ausgewählt aus der Liste bestehend aus einer der SEQ ID NO: 18-75 oder bestehend aus den spezifischen Teilsätzen von Peptiden, wie in einem der Ansprüche 3 oder 4 definiert werden, zu erkennen;
b. einen Träger, in welchem die Fänger-Biomoleküle aus Schritt a) immobilisiert werden; und
c. ein zweites Erkennungsmolekül (*detection biomolecule*)*,* welches in der Lage ist, den Zielanalyten oder das Fänger-Biomolekül, welches wahlweise an einem Markierungsmolekül gebunden ist, zu erkennen;
zur Prognose der Anzahl von Reaktionen während der oralen Immuntherapie (OIT) gegen Proteine in Kuhmilch und/oder einer Schätzung der Behandlungszeit, welche benötigt wird, um Toleranz oder Desensibilisierung während der OIT zu erreichen, und/oder des Bedürfnisses Prämedikation während der OIT anzuwenden.

## Revendications

1. Procédé de pronostic d'immunothérapie orale (OIT) contre les protéines du lait de vache dans un sujet humain incapable de tolérer le lait de vache, comprenant la détermination de la présence ou absence d'anticorps IgE dans un échantillon biologique isolé à partir du sujet prélevé avant de commencer le traitement par OIT (temps 0), tel que le sang, le sérum, le plasma, le liquide céphalo-rachidien, le liquide péritonéal, les selles ou l'urine, capables de reconnaître des peptides identifiés avec l'une quelconque des séquences suivantes :
a. au moins une séquence choisie parmi le groupe composé des séquences SEQ ID NO : 18-32 ; et/ou
b. au moins une séquence choisie parmi le groupe composé des séquences SEQ ID NO : 33-45 ; et/ou
c. au moins une séquence choisie parmi le groupe composé des séquences SEQ ID NO : 46-63 ; et/ou
d. au moins une séquence choisie parmi le groupe composé des séquences SEQ ID NO : 64-69 ; et/ou
e. au moins une séquence choisie parmi le groupe composé des séquences SEQ ID NO : 70-75;
dans lequel le pronostic d'immunothérapie orale (OIT) contre les protéines du lait de vache est établie par corrélation du nombre de séquences peptidiques reconnues par les anticorps IgE dans l'échantillon du sujet avant de commencer le traitement par OIT (temps 0) avec des valeurs de référence établies sur la base de ces mêmes séquences peptidiques qui mettent en corrélation le nombre de peptides reconnus par les anticorps IgE dans les échantillons des patients avec la réponse après le traitement par OIT.

2. Procédé selon la revendication 1, où ledit procédé est un procédé de pronostic du nombre de réactions pendant l'immunothérapie orale (OIT) et la détermination de la présence ou absence d'anticorps IgE dans l'échantillon biologique, dans lequel ledit procédé est effectué par la détermination de la présence ou absence d'anticorps IgE capables de reconnaitre des peptides identifiés avec l'une quelconque des séquences suivantes :
a. séquences SEQ ID NO : 19, 21, 25 et 27 ; et
b. séquences SEQ ID NO : 36, 37 et 38; et
c. séquences SEQ ID NO : 51, 53, 58, 59 et 62; et
d. séquence SEQ ID NO : 69 ; et
e. séquences SEQ ID NO : 71 et 72.

3. Procédé selon la revendication 1, dans lequel le procédé est un procédé de pronostic d'une estimation du temps de traitement nécessaire pour parvenir à la tolérance ou la désensibilisation pendant OIT et la détermination de la présence ou absence d'anticorps IgE dans l'échantillon biologique, dans lequel le procédé est effectué par la détermination de la présence ou absence d'anticorps IgE capables de reconnaitre des peptides identifiés avec l'une quelconque des séquences suivantes :
a. séquences SEQ ID NO : 28 et 31 ; et
b. séquences SEQ ID NO : 36-40 et 44; et
c. séquences SEQ ID NO : 53, 55 et 56; et
d. séquences SEQ ID NO : 67 et 68; et
e. séquences SEQ ID NO : 70, 72 et 75.

4. Procédé selon la revendication 1, où la détermination de la présence ou absence d'anticorps IgE dans l'échantillon biologique est effectuée par la détermination de la présence ou absence d'anticorps IgE capables de reconnaitre des peptides identifiés avec les séquences suivantes : SEQ ID NO : 18-75, dans lequel :
a. une reconnaissance de peptides de moins de 15% desdites séquences est indicative d'un bon pronostic, dans lequel le bon pronostic fait référence à une probabilité faible d'avoir des réactions indésirables pendant la désensibilisation (<7 réactions) et de nécessiter un délai court pour parvenir à la tolérance au lait de vache (<8 semaines) et de ne pas nécessiter de prémédication ; une reconnaissance de peptides d'entre 15%-75% desdites séquences est indicative d'un pronostic modéré, dans lequel le pronostic modéré fait référence à un nombre élevé de réactions allergiques pendant la désensibilisation (>7 réactions) ou à nécessiter un long délai pour parvenir à la tolérance au lait de vache (>8 semaines) ou à nécessiter des anti-histamines comme prémédication ; et
b. une reconnaissance de peptides de plus de 75% desdites séquences est indicative d'un pronostic pauvre, dans lequel le pronostic pauvre fait référence à un nombre élevé de réactions allergiques pendant la désensibilisation (>7 réactions) et à nécessiter un long délai pour parvenir à la tolérance au lait de vache (>8 semaines) et à nécessiter des anti-histamines et des corticoïdes comme prémédication.

5. Procédé selon l'une quelconque des revendications précédentes, dans lequel la reconnaissance de peptides est déterminée par le biais d'un immunoessai.

6. Procédé selon l'une quelconque des revendications 1 à 4, dans lequel la reconnaissance de peptides est déterminée par le biais de micro-réseaux peptidiques.

7. Utilisation d'un micro-réseau peptidique approprié pour mettre en œuvre le procédé selon l'une quelconque des revendications 1-4 comprenant les combinaisons de peptides selon l'une quelconque des revendications 1-4, pour le pronostic de l'immunothérapie orale (OIT) contre les protéines du lait de vache dans un sujet humain.

8. Utilisation *in vitro* d'une trousse comprenant des peptides comme biomolécules de capture ; dans lequel lesdits peptides sont choisis parmi la liste composée de l'une quelconque des SEQ ID NO : 18-75, pour le pronostic du nombre de réactions pendant l'immunothérapie orale (OIT) contre les protéines du lait de vache et/ou une estimation du temps de traitement nécessaire pour parvenir à la tolérance ou désensibilisation pendant l'OIT et/ou la nécessité d'appliquer de la prémédication pendant l'OIT.

9. Utilisation *in vitro* d'une trousse comprenant :
a. des biomolécules aptes à reconnaître des IgE choisies parmi la liste composée de l'une quelconque des SEQ ID NO : 18-75 ou composée de sous-ensembles spécifiques de peptides selon l'une quelconque des revendications 3 ou 4 ; et
b. une seconde molécule de reconnaissance (biomolécule de détection) apte à reconnaître l'analyte cible ou la biomolécule de capture reliée optionnellement à une molécule étiquette ;
pour le pronostic du nombre de réactions pendant l'immunothérapie orale (OIT) contre les protéines du lait de vache et/ou une estimation du temps de traitement nécessaire pour parvenir à la tolérance ou la désensibilisation pendant l'OIT et/ou la nécessité d'appliquer de la prémédication pendant l'OIT.

10. Utilisation d'une trousse comprenant :
a. des biomolécules de capture aptes à reconnaître des IgE choisies parmi la liste composée de l'une quelconque des SEQ ID NO : 18-75 ou composée de sous-ensembles spécifiques de peptides selon l'une quelconque des revendications 3 ou 4 ;
b. un support où les biomolécules de capture de l'étape a) sont immobilisés ; et
c. une seconde molécule de reconnaissance (biomolécule de détection) apte à reconnaître l'analyte cible ou la biomolécule de capture reliée optionnellement à une molécule étiquette ;
pour le pronostic du nombre de réactions pendant l'immunothérapie orale (OIT) contre les protéines du lait de vache et/ou une estimation du temps de traitement nécessaire pour parvenir à la tolérance ou la désensibilisation pendant l'OIT et/ou la nécessité d'appliquer de la prémédication pendant l'OIT.
